# EUROPÄISCHE PATENTANMELDUNG

(11) **EP 4 759 915 A2**
(43) Veröffentlichungstag der Anmeldung: **17.06.2026**
(21) Anmeldenummer: 25209807.4
(22) Anmeldetag: 20.10.2025
(51) Int. Cl.: C12M 1/00

(54) **PHOTOBIOREAKTOR ZUR SUBMERSEN PRODUKTION VON TORFMOOS**

(30) Priorität: 18.11.2024 DE 102024133668
(71) Anmelder: Hochschule Anhalt, Körperschaft des öffentlichen Rechts, 06366 Köthen (DE)
(72) Erfinder: Grewe, Claudia, 06369 Prosigk (DE); Glaubitz, Maria, 06112 Halle (Saale) (DE); Posten, Clemens, 76139 Karlsruhe (DE)
(74) Vertreter: Gulde & Partner

(57) **Zusammenfassung**

Die Erfindung betrifft einen Photobioreaktor (1) zur submersen Produktion von Torfmoos umfassend einen Behälter (10) mit einem Reaktorinnenraum (RI) mit zumindest einem Teil des Reaktorinnenraums (RI). Der Behälter (10) weist ein erstes Kompartiment (11) und ein über dem ersten Kompartiment (11) angeordnetes zweites Kompartiment (12) auf, wobei die Kompartimente (11, 12, 13) den zumindest einen Teil des Reaktorinnenraums (RI) ausbilden, im Wesentlichen transparent sind und ein Verhältnis von Höhe (H) zu Durchmesser (D) jedes Kompartiments 1 bis 2,6 beträgt. Ferner weist der Behälter (10) ein zwischen dem ersten und dem zweiten Kompartiment (11, 12) angeordnetes Trennelement (14) aufweisend eine Vielzahl von Öffnungen (141) zur fluidleitenden Verbindung von den Kompartimenten (11, 12, 13) sowie eine verschließbare Zugriffsöffnung zur Zugabe und Entnahme von mineralischem Nährmedium und Torfmoos-Gametophyten in den bzw. aus dem zumindest einen Teil des Reaktorinnenraums (RI) auf. Zudem umfasst der Behälter (10) einen in einem unteren Bereich (II) des Reaktorinnenraums (RI) mittig angeordneten Gasverteiler (15) mit einer Vielzahl von Gasöffnungen zur Zufuhr von Gas in den zumindest einen Teil des Reaktorinnenraums (RI) entlang eines begasten Teilbereichs einer Photobioreaktorquerschnittsfläche, und einen in einem oberen Bereich (I) des Reaktorinnenraums (RI) angeordneten Gasauslass (16) zur Abfuhr von Abgas aus dem Behälter (10). Die Erfindung betrifft ferner ein Verfahren zur submersen Produktion von Torfmoos, welches mittels eines erfindungsgemäßen Photobioreaktors (1) durchgeführt wird.

## Beschreibung

Die Erfindung betrifft einen Photobioreaktor zur submersen Produktion von Torfmoos. Die vorliegende Erfindung betrifft ferner ein Verfahren zur submersen Produktion von Torfmoos, welches mittels eines erfindungsgemäßen Photobioreaktors durchgeführt wird.

### Technologischer Hintergrund

Torfmoose (Sphagnum) können in Photobioreaktoren erheblich schneller vermehrt werden als im Freiland und werden dringend kostengünstig in großer Menge als Saatgut für Torfmoospaludikulturen benötigt. Der Photobioreaktorprozess zur Torfmoosproduktion basiert auf dem Prinzip der ständigen Bereitstellung von Licht, CO₂ und mineralischen Nährstoffen in einem abgeschlossenen, regelbaren Reaktorsystem. Dabei ist die Biomasseproduktivität (Wachstumsrate und Konzentration) in einem Photobioreaktor ein ausschlaggebendes Auswahlkriterium zur Umsetzung einer Torfmoosvermehrung, da das Produkt Torfmoos in möglichst großer Menge, bezogen auf das Volumen und die Zeiteinheit sowie die eingesetzte Energie, bereitgestellt werden soll.

Aktuell findet die Torfmoosproduktion im Photobioreaktor nur innerhalb universitärer Einrichtungen im Labormaßstab statt, wobei standardmäßig unter sterilen Bedingungen gearbeitet wird. Hierfür kommen Rührreaktoren mit Volumina von 5 bis 12 Liter zum Einsatz (Decker & Reski, 2020), weshalb in ihnen nur kleine Mengen Torfmoos produziert werden können. Zudem sind die Investitionskosten sowie die spezifischen Kosten pro Liter Torfmooskultur aufgrund des kleinen Maßstabs und der Steriltechnik sehr hoch. Es existieren weitere, kostengünstige Versuche, Torfmoos im Photobioreaktor zu kultivieren, wobei entweder emers oder submers-flotierend kultiviert wird (Melková, 2022). Dies führt jedoch zu Einbußen der Biomasseproduktivität, da durch fehlende Konvektion und Homogenität im Photobioreaktor Nährstoffe und Licht ungleichmäßig zur Verfügung gestellt werden. Das technische Problem ist also, eine kostengünstige Skalierung des Reaktorvolumens zu erreichen. Dies ist aber aufgrund der beschränkten Lichteindringtiefe, der ungleichmäßigen Verteilung der Nährstoffe, sowie der Flotationsneigung der Gametophyten schwierig.

Die Torfmooskultivierung im Photobioreaktor wurde erstmals durch Rudolph et al. (1988) im Labormaßstab gezeigt, Hier wurde in einem 2 Liter großen, belichteten und begasten Rundkolben kultiviert. Des Weiteren wurde ein Photobioreaktorprozess in einem 7-Liter Doppelwandrundkolben beschrieben, welcher die Kultivierung von Torfmoosen und anderen Laub- und Lebermoosen umfasst (Reski and Gorr, 1999). Das Verfahren beschreibt die axenische Vermehrung der Moose unter Verwendung preisintensiver Steriltechnik und ist deshalb zur Massenvermehrung im landwirtschaftlichen Kontext ungeeignet. In Veröffentlichungen der Universität Freiburg werden auch Rührreaktoren zur Kultivierung eingesetzt (Beike et al., 2015; Heck et al., 2021). Der Maßstab beträgt hier 5 Liter bis 12 Liter, die Skalierbarkeit der technisch komplexeren Rührreaktoren ist für phototrophe Prozesse jedoch begrenzt und der Bioreaktormaßstab dieser genannten Prozesse ist zur Massenproduktion von Torfmoos um Größenordnungen zu gering. In der Dissertationsschrift von Melková (2022) wird die Vermehrung des Torfmooses ebenfalls im Labormaßstab in einem sogenannten Wave-Photobioreaktor (3 Liter) beschrieben und basiert jedoch auf dem Single-Use Prinzip und ist zur Verwendung zur Torfmoosmassenproduktion aus Materialkostengründen und dem Anspruch der Sparsamkeit mit Rohstoffen zur Reaktorherstellung nicht angemessen. Auch Niederkrüger et al. (2014) beschreiben Single-Use Wave-Photobioreaktoren. Ein Photobioreaktorkonzept von Wright (2024) beschreibt 2 Liter und 5 Liter Eimer mit Gaszufuhr im Kopfraum. Ferner wurde Torfmoos in der Arbeit von Melková (2022) in einem 1,5 Liter Moosbettreaktor kultiviert, in welchem sich die Biomasse emers in einem abgeschlossenen und mit Luft, CO₂ und Nährmedium versorgten Behälter vermehrt.

Funktionsfähige Photobioreaktoren, die zur Vermehrung von Sphagnum in der Größenordnung von m³ arbeiten, sind bislang nicht beschrieben.

### Zusammenfassung der Erfindung

Die erfindungsgemäße Aufgabe wird gelöst durch die Gegenstände der unabhängigen Patentansprüche. Bevorzugte Weiterbildungen sind Gegenstand der Unteransprüche.

Ein erster Aspekt der vorliegenden Offenbarung betrifft einen Photobioreaktor zur submersen Produktion von Torfmoos umfassend einen Behälter mit einem Reaktorinnenraum mit zumindest einem Teil des Reaktorinnenraums zur Aufnahme von einem mineralischen Nährmedium und Torfmoos-Gametophyten. Der Behälter weist ein erstes Kompartiment und ein über dem ersten Kompartiment angeordnetes zweites Kompartiment auf, wobei die Kompartimente den zumindest einen Teil des Reaktorinnenraums ausbilden, wobei die Kompartimente - zur Einkopplung von eingestrahltem Licht in den zumindest einen Teil des Reaktorinnenraums - im Wesentlichen transparent sind und ein Verhältnis von Höhe zu Durchmesser jedes Kompartiments 1 bis 2,6 beträgt. Ferner weist der Behälter ein zwischen dem ersten und dem zweiten Kompartiment angeordnetes Trennelement, eine Vielzahl von Öffnungen zur fluidleitenden Verbindung von den Kompartimenten sowie eine verschließbare Zugriffsöffnung zur Zugabe und Entnahme von mineralischem Nährmedium und Torfmoos-Gametophyten in den bzw. aus dem zumindest einen Teil des Reaktorinnenraums (und zu dessen Reinigung) auf. Zudem umfasst der Behälter einen in einem unteren Bereich des Reaktorinnenraums mittig angeordneten Gasverteiler mit einer Vielzahl von Gasöffnungen zur Zufuhr von Gas in den zumindest einen Teil des Reaktorinnenraums entlang eines begasten Teilbereichs einer Photobioreaktorquerschnittsfläche, und einen in einem oberen Bereich des Reaktorinnenraums angeordneten Gasauslass zur Abfuhr von Abgas aus dem Behälter.

Mit anderen Worten wird ein Photobioreaktor bereitgestellt, der vorzugsweise über einen Behälter verfügt, wobei der Behälter einen abgeschlossenen fluidleitend verbundenen (in sich geschlossenen und fluidleitend verbundenen) Reaktorinnenraum aufweist, der über die Zugriffsöffnung und den Gasauslass zugänglich bzw. steuerbar ist. Der zumindest eine Teil des Reaktorinnenraums ist vorzugsweise ausgebildet, ein flüssiges Nährmedium, im weiteren auch als Flüssigphase bezeichnet, mit Mineralsalzen und Torfmoos-Gametophyten aufzunehmen.

Das erste Kompartiment und das zweite Kompartiment sind mit anderen Worten vorzugsweise entlang einer vertikalen Richtung angeordnet, wobei das zweite Kompartiment auf dem ersten Kompartiment bzw. oberhalb des ersten Kompartiments angeordnet ist. "Auf" bedeutet im Rahmen der vorliegenden Erfindung vorzugsweise sowohl eine direkte (unmittelbare) Anordnung eines Kompartiments auf dem anderen Kompartiment, lediglich mit dem Trennelement zwischen den beiden Kompartimenten und keinem weiteren Kompartiment, als auch eine indirekte (mittelbare) Anordnung des einen Kompartiments auf dem anderen Kompartiment, bei der weitere Kompartimente zusätzlich zu dem Trennelement zwischen dem einen und dem anderen Kompartiment vorhanden sein können. Eine direkte Anordnung oder ein (direktes) Angrenzen von Kompartimenten ist demnach im Rahmen der vorliegenden Erfindung vorzugsweise so zu verstehen, dass keine weiteren Kompartimente zwischen den direkt aufeinander (unmittelbar benachbart) angeordneten bzw. zwischen den direkt einander angrenzenden Kompartimenten vorhanden sind.

Die Kompartimente bzw. ein Verbund der Kompartimente bilden vorzugsweise den zumindest einen Teil des Reaktorinnenraums aus. Mit anderen Worten weisen die Kompartimente jeweils eigene Reaktorräume bzw. Kultivierungsräume auf, die kumuliert den zumindest einen Teil des Reaktorinnenraums ausbilden. Die Kompartimente sind demnach mit anderen Worten fluidleitend miteinander verbunden, sodass zumindest ein Austausch von in ihnen befindlichem flüssigem Nährmedium erfolgen kann.

Die Kompartimente sind im Wesentlichen transparent, ihre Wände umfassen also ein transparentes Material oder bestehen aus einem transparenten Material, das lichtdurchlässig ist, sodass Nährmedium und Gametophyten mit Licht bestrahlt werden können. Der Begriff "im wesentlichen transparent" bezeichnet vorliegend vorzugsweise Kompartimente, die in einem Maße lichtdurchlässig ist, dass dahinterliegende Objekte oder Strukturen zumindest teilweise erkennbar sind beziehungsweise eintretendes Licht empfangen können. Der Begriff umfasst auch Kompartimente, die geringfügige Trübungen, Farbnuancen oder Oberflächenstrukturen aufweisen, solange deren optische Eigenschaften für den vorgesehenen Verwendungszweck eine überwiegende Lichtdurchlässigkeit ermöglichen. Eine absolute Transparenz der Kompartimente ist hierbei besonders bevorzugt. Dabei ist im Rahmen der vorliegenden Erfindung unter im Wesentlichen transparent ferner bevorzugt zu verstehen, dass ein wesentlicher Teil, vorzugsweise mindestens 90%, der (formgebenden) Flächen (Wände) der Kompartimente transparent sind und entsprechende kleine zusätzliche intransparente Bauteile wie Montageelemente, Sensoren, Sonden usw. dieser Eigenschaft nicht grundsätzlich widersprechen. Hierdurch ist also eine Bestrahlung und Einkopplung von Licht in den zumindest einen Teil des Reaktorinnenraums möglich.

Der Begriff "im Wesentlichen transparent" bezeichnet vorliegend vorzugsweise Kompartimente, die eine Lichttransmission im sichtbaren Spektralbereich (400 - 700 nm) von mindestens 70 %, vorzugsweise mindestens 80 %, aufweisen, wobei eine geringe Trübung (Haze) von beispielsweise bis zu 10 % toleriert wird. Eine vollständige oder absolute Transparenz ist hierbei nicht erforderlich, sofern die optischen Eigenschaften für den vorgesehenen Verwendungszweck ausreichend sind.

Der Begriff "absolut transparent" bezeichnet vorliegend vorzugsweise Kompartimente mit einer Lichttransmission im sichtbaren Spektralbereich (400 - 700 nm) von mindestens 90 %, vorzugsweise mindestens 95 %, insbesondere gemessen nach ASTM D1003 oder einer vergleichbaren Norm.

Das Trennelement unterteilt mit anderen Worten vorzugsweise den von den Kompartimenten ausgebildeten Teil des Reaktorinnenraums, sodass zumindest zwei Kultivierungskammern bzw. - räume innerhalb des Teils des Reaktorinnenraums gebildet werden. Gleichzeitig bleibt ein Austausch einer flüssigen Phase aufgrund der Vielzahl von Öffnungen des Trennelements zwischen den einzelnen Kultivierungskammern möglich.

Mit Hilfe der Zugriffsöffnung wird die Zugabe der notwendigen Bestandteile für die Produktion von Torfmoos ermöglicht. Um den abgeschlossen Reaktorinnenraum auszubilden, ist die Zugriffsöffnung vorzugsweise öffenbar und verschließbar. Auch die Reinigung des Behälters und der Kompartimente ist mittels der Zugriffsöffnung möglich. Die Zugriffsöffnung ist vorzugsweise im unteren oder oberen Bereich des Reaktorinnenraums angeordnet.

Unter dem "unteren Bereich" des Reaktorinnenraums wird vorzugsweise ein Bereich verstanden, der sich im unteren Drittel, insbesondere im unteren Viertel, einer Höhe (vorzugsweise einer Innenraumhöhe) des Reaktorinnenraums befindet. Unter dem "oberen Bereich" des Reaktorinnenraums wird vorzugsweise ein Bereich verstanden, der sich im oberen Drittel, insbesondere im oberen Viertel, der Höhe (vorzugsweise der Innenraumhöhe) befindet.

Der Gasverteiler ist mit anderen Worten vorzugsweise ausgebildet, Gase in den Reaktorinnenraum einzuleiten, und der Gasauslass ist vorzugsweise ausgebildet, gasförmige Produkte, im Weiteren auch als Abgas bezeichnet, aus dem Reaktorinnenraum herauszuleiten.

Der Gasverteiler dient dazu, das eingeleitete Gas mit der Suspension umfassend das Nährmedium als Flüssigphase und die Gametophyten als Feststoffphase in Kontakt zu bringen. Dabei wird vorzugsweise ein Gasstrom in der flüssigen Phase in Form von Blasen dispergiert. **Im** Schwerefeld sorgt der Dichteunterschied zwischen der am Boden dispergierten Gasphase und der Flüssigphase für die Durchmischung des Reaktionsmediums durch die aufsteigenden Gasblasen. Gleichzeitig erfolgt der Stofftransport gasförmig-flüssig über die Phasengrenze zwischen Gasblasen und Flüssigphase. Der Gasverteiler ist mit anderen Worten in einem mittigen bzw. zentralen Bereich eines Querschnitts des Reaktorinnenraums angeordnet, sodass ein umlaufender Randbereich freibleibt. Der Gasverteiler ist vorzugsweise in dem (innerhalb des) zumindest einen Teil des Reaktorinnenraums angeordnet.

Der zumindest eine Teil des Reaktorinnenraums ist vorzugsweise ein Bestandteil bzw. Teilbereich/- volumen des Reaktorinnenraums und ist daher mit dem verbleibenden Teil des Reaktorinnenraums fluidleitend verbunden.

Die Kompartimente besitzen vorzugsweise jeweils eine erste Dimension in einer horizontalen Richtung, die einer Stelle des Kompartiments mit dem höchsten Betrag bzw. Wert dieser Dimension entspricht, also mit anderen Worten einem breitesten Bereich des Kompartiments entspricht, der durch die Wände des Kompartiments begrenzt wird. Diese erste Dimension bzw. ihr Wert entspricht vorzugsweise in dem Verhältnis von Höhe zu Durchmesser dem Durchmesser. Ferner besitzen die Kompartimente vorzugsweise jeweils eine zweite Dimension in einer vertikalen Richtung, die einer Stelle des Kompartiments mit dem höchsten Betrag bzw. Wert dieser Dimension entspricht, also mit anderen Worten einem höchsten Bereich des Kompartiments entspricht. der durch die Wände des Kompartiments begrenzt wird. Sofern die Kompartimente an ihren einander gegenüberliegenden Enden Öffnungen zur fluidleitenden Verbindung aufweisen, bilden diese Öffnungen die für die Dimensionen maßgeblichen Begrenzungen. Diese zweite Dimension bzw. ihr Wert entspricht vorzugsweise in dem Verhältnis von Höhe zu Durchmesser der Höhe. Die verschiedenen Kompartimente können identische Dimensionen oder verschiedene Dimensionen ebenso wie identische oder verschiedene Verhältnisse von Höhe zu Durchmesser aufweisen, sofern diese innerhalb des besagten Bereichs des Verhältnisses liegen. Mit anderen Worten können sich die Durchmesser verschiedener Kompartimente auch unterscheiden, genauso wie die jeweilige Höhe.

Die Photobioreaktorquerschnittsfläche ist vorzugsweise die Querschnittsfläche des Kompartiments in einer Ebene, die senkrecht zur vertikalen Richtung verläuft, und entspricht insbesondere der Fläche an dem breitesten Bereich des Kompartiments senkrecht zur vertikalen Richtung. Bei Kompartimenten mit unveränderlichem Querschnitt ist somit die Photobioreaktorquerschnittsfläche gleichbleibend. Mit anderen Worten: Bei Kompartimenten mit konstantem Querschnitt entspricht die Photobioreaktorquerschnittsfläche diesem konstanten Querschnitt.

Demnach ist der Gasverteiler vorzugsweise ausgebildet, eine flächige Verteilung von zugeführtem Gas bereitzustellen, wobei das Gas entlang des begasten Teilbereichs der Photobioreaktorquerschnittsfläche zugeführt wird und wobei entlang eines verbleibenden unbegasten Teilbereichs der Photobioreaktorquerschnittsfläche keine Zufuhr von Gas erfolgt. Die Photobioreaktorquerschnittsfläche entspricht vorzugsweise der Querschnittsfläche des zumindest einen Teil des Reaktorinnenraums bzw. des Reaktorinnenraums senkrecht zur vertikalen Achse des Photobioreaktors. Selbstverständlich erfolgt stromabwärts des zugeführten Gases auch eine Quervermischung, die nicht ausgeschlossen werden kann, sodass auch ursprünglich unbegaste nachfolgende Teilbereiche einer Gaszufuhr unterworfen sein können. Vorliegend wird jedoch die Zufuhr bzgl. einer Querschnittsfläche senkrecht zu einer vertikalen Richtung des Photobioreaktors als Bezugsfläche betrachtet. Mit anderen Worten definiert der Gasverteiler vorzugsweise durch seine Gasöffnungen in einer horizontalen Ebene, die in einem Querschnitt des Reaktorinnenraums liegt, der senkrecht zur vertikalen Achse des Photobioreaktors verläuft, eine Fläche des begasten Teilbereichs, die nur einen Teil des gesamten Querschnitts des Reaktorinnenraums ausmacht. Diese Fläche des begasten Teilbereichs wird vorzugsweise definiert als die Fläche, die durch sämtliche Gasöffnungen, aus denen Gas strömen kann, umspannt wird. Die Fläche kann rechteckförmig, kreisförmig, ellipsenförmig, kreisringförmig oder dreieckig sein oder weitere Formen annehmen. Die entsprechende Anordnung der Gasöffnungen ist demnach ebenfalls rechteckförmig, kreisförmig, ellipsenförmig, kreisringförmig oder dreieckig usw. Kreisförmig bedeutet dabei, dass eine Fläche des gesamten Kreises mit Öffnungen versehen ist, wohingegen kreisringförmig bedeutet, dass nur eine Fläche entlang eines Umfangs bzw. eines Umfangs mit einer definierten Breite mit Öffnungen versehen ist. Dabei werden mit anderen Worten vorzugsweise die einzelnen Gasöffnungen des Gasverteilers fiktiv miteinander verbunden, wobei einander am nächsten liegende (den geringsten Abstand zueinander besitzende) Gasöffnungen miteinander verbunden werden, wobei die resultierende Form der fiktiv verbundenen Gasöffnungen den begasten Teilbereich bzw. dessen Flächenform bildet. Diese Definition entspricht demzufolge nicht der effektiven Fläche der Gasöffnungen (mit anderen Worten des Gasaustrittsfläche) selbst, durch die das Gas austritt, die mit anderen Worten die kumulierte Fläche der Gasöffnungen darstellt. Die kumulierte Fläche der Gasöffnungen ist vorzugsweise niedriger als die Fläche des begasten Teilbereichs. Hierdurch wird sichergestellt, dass ein Bereich des Reaktorinnenraums begast wird und ein anderer Bereich nicht begast wird. Da die mittlere Dichte des im entsprechenden Teil des Reaktorinnenraums befindlichen Mediums mit dispergierter Gasphase geringer ist als die mittlere Dichte des Mediums im unbegasten Teil, stellt sich im Schwerefeld eine Umlaufströmung (Zirkulationsströmung) der flüssigen Phase ein. Da Gasblasen bevorzugt in der Säulenmitte aufsteigen, entsteht ein radiales Geschwindigkeitsprofil mit einem hohen Gasgehalt in Achsennähe im Vergleich zu den wandnahen Bereichen. Die resultierende Dichtedifferenz bewirkt eine abwärtsgerichtete Strömung in Wandnähe und eine aufwärtsgerichtete in der Mitte des Behälters. Hierdurch bildet sich eine Zellenstruktur in dem Photobioreaktor aus. Hierdurch wird eine radiale Zirkulationsströmung erzielt, unter anderem hervorgerufen durch die - bei relativer Betrachtung - Dichteunterschiede zwischen Bereichen, in denen ein hoher Gasanteil durch die Gaszufuhr vorliegt, und Bereichen, in den ein geringer Gasanteil vorliegt. Es resultieren Dichteunterschiede, aufgrund derer eine radial zirkuläre Strömung entsteht. Diese ist aufwärts entlang einer vertikalen Achse des Photobioreaktors, radial in Richtung der Behälterwand, abwärts entlang der Behälterwand und schließlich radial in Richtung einer Mitte des Photobioreaktors gerichtet.

Die mittige Anordnung des Gasverteilers bedeutet mit anderen Worten eine Anordnung in einem zentral-axialen Bereich des Reaktorinnenraums. Der Gasverteiler ist vorzugsweise über eine fluidleitende Verbindung bzw. Verbindungsleitung mit einer externen Gasquelle verbunden.

Der Begriff "mittig" bezeichnet vorliegend vorzugsweise eine Anordnung im zentralen Bereich des Reaktorinnenraums, vorzugsweise entlang der Längsachse bzw. Zentralachse des Reaktors. "Mittig" bezeichnet besonders bevorzugt eine Position innerhalb von 70 % des radialen Abstands von der Zentralachse des Reaktorinnenraums zu dessen seitlichen Wandungen.

Mit Hilfe des erfindungsgemäßen Photobioreaktors kann die produktive, suspendierte Torfmoos-Kultivierungsform in kostengünstigen, technischen Behältern durchgeführt werden. Es kann ein Volumen von zwei- und dreistelligen Literbereichen zur Torfmoosproduktion genutzt werden, wobei die Skalierbarkeit durch eine Erhöhung von Durchmesser und Höhe des Photobioreaktors ermöglicht wird. Der Photobioreaktor verfügt über einen einfachen und robusten Aufbau und benötigt zur Durchmischung lediglich pneumatischen Leistungseintrag, was die kostengünstige Bereitstellung von Torfmoos ermöglicht. Durch die innovative Form von Behälter und Trennelement wird in Kombination mit der Begasung (volumetrische Gaslast) eine vollständige und dauerhafte Suspendierung der Torfmoos-Gametophyten ermöglicht. Durch das Zusammenspiel der Form des Photobioreaktors und der Begasung (zentral-axial) entstehen radiale zirkuläre Strömungen, die die Torfmoose regelmäßig an der hellen Innenseite des Reaktorinnenraums vorbeiführen und so Limitierungen vermeiden auch bei Durchmessern, die die Länge des Lichtpfades wesentlich überschreiten. Dies ermöglicht gleichzeitig den Einsatz geringer CO₂-Anteile, wie zum Beispiel durch Verwendung von Umgebungsluft, in der Zuluft und eine optimierte Gasnutzung, welche den Austrag von ungenutztem CO₂ reduziert oder minimiert. Bei Verwendung von Umgebungsluft wird dem Reaktor vorzugsweise verdichtete Umgebungsluft als Druckluft zugeführt. Der Anteil von CO₂ in Umgebungsluft beträgt etwa 0,04 Vol.-%. Vorzugsweise erfolgt aber eine Beimischung von CO₂ zur Umgebungsluft, sodass die Reaktor-Zuluft 1 - 2 Vol.-% CO₂ enthält. Insgesamt sind der spezifische Leistungseintrag und die resultierenden Scherkräfte gering, um einerseits eine kosteneffiziente Durchmischung des Reaktorinhalts zu erreichen und andererseits die makroskopischen Flocken von Torfmoos und damit die morphologische Struktur der Gametophyten nicht zu beeinträchtigen. Ferner ist der Behälter bzw. die Kompartimente wiederverwendbar, bei gleichzeitig verbessertem Strömungsprofil, verbesserter Durchmischung und einem hohen Ausmaß an CO₂-Aufnahme zur Verhinderung von Emissionen.

**Im** Numbering-up können Volumina im m³-Maßstab erreicht werden. Eine Skalierung in die Höhe gelingt vorzugsweise durch die Abtrennung einzelner Kompartimente mittels des Trennelements, um eine Flotation entlang der vertikalen Achse zu vermeiden. Hierdurch werden die eingangs beschriebenen Mechanismen innerhalb der einzelnen Kompartimente beibehalten.

Vorzugsweise entspricht der zumindest eine Teil des Reaktorinnenraums dem gesamten Reaktorinnenraum. Vorzugsweise bilden die Kompartimente den gesamten Reaktorinnenraum aus.

Die unmittelbar benachbarten Kompartimente sind bevorzugt stoffschlüssig, form- und/oder kraftschlüssig miteinander verbunden. Das Trennelement ist vorzugsweise innerhalb eines Verbindungsbereichs direkt benachbarter bzw. angrenzender Kompartimente angeordnet. Der Begriff "Verbindungsbereich" bezeichnet vorliegend vorzugsweise denjenigen Bereich, in dem zwei unmittelbar benachbarte oder angrenzende Kompartimente stoffschlüssig, formschlüssig und/oder kraftschlüssig miteinander verbunden sind. Dieser Bereich umfasst vorzugsweise aneinandergrenzende Wandabschnitte der Kompartimente und kann insbesondere Schweißnähte, Fügestellen oder mechanische Verbindungselemente einschließen.

Vorzugsweise umfasst der Photobioreaktor eine Lichtquelle zur Erzeugung des Lichts.

Der Photobioreaktor umfasst vorzugsweise keine mechanisch bewegten Einbauten. Es liegt also eine vollständige Abwesenheit von mechanisch bewegten Einbauten vor, wie sie beispielsweise in Form von Rührwerken, Pumpen, Schaufelrädern oder anderen innerhalb des Reaktorinnenraums angeordneten beweglichen Komponenten auftreten könnten. Dadurch wird der Betrieb des Reaktors vereinfacht, die Anfälligkeit für mechanischen Verschleiß verringert und das Risiko von Kontaminationen reduziert, die durch bewegliche Teile hervorgerufen werden können. Zudem ermöglicht diese Ausgestaltung einen energieeffizienteren Betrieb, da keine zusätzliche Energie für den Antrieb mechanischer Elemente erforderlich ist.

In einer bevorzugten Ausgestaltung beträgt ein Verhältnis von einer Fläche des begasten Teilbereichs zu einer Fläche des unbegasten Teilbereichs 0,1 bis 1,0 vorzugsweise 0,2 bis 0,7 und besonders bevorzugt 0,3 bis 0,5. Hierdurch wird einerseits sichergestellt, dass entlang des Querschnitts ausreichend Dichteunterschiede für die Ausbildung der radialen Zirkulationsströmung vorliegen und gleichzeitig eine hinreichende Zufuhr von Gas, insbesondere Luft erfolgt, sodass ausreichend CO₂ zugeführt wird, und andererseits der Austrag an unverbrauchtem CO₂ reduziert bzw. verhindert wird. Zudem wird eine ausreichend starke Strömung für eine verbesserte Dispersion der Gametophyten sichergestellt.

In einer bevorzugten Ausgestaltung beträgt die kumulierte Fläche der Öffnungen des Gasverteilers 5 bis 60 %, vorzugsweise 10 bis 50 % und besonders bevorzugt 15 bis 40 % der Photobioreaktorquerschnittsfläche. Hierdurch ist es möglich, dass auch flächige, poröse Sparger mit vergleichbarem Durchmesser benutzt werden können.

In einer ebenfalls bevorzugten Ausführungsform bildet das erste Kompartiment den unteren Bereich des Reaktorinnenraums aus und das zweite Kompartiment bildet den oberen Bereich des Reaktorinnenraums aus. Mit anderen Worten ist das erste Kompartiment in dem unteren Bereich des Reaktorinnenraums angeordnet und bildet einen Boden des Behälters aus und das zweite Kompartiment ist in dem oberen Bereich des Reaktorinnenraums angeordnet und bildet einen Deckel des Behälters aus.

Vorzugsweise umfasst das erste Kompartiment den Gasverteiler. Vorzugsweise ist der Gasverteiler im ersten Kompartiment oder auf dem Boden des ersten Kompartiments angeordnet. Dieser ist mit anderen Worten in diesem Fall auf einem Boden des ersten Kompartiments angeordnet und über eine fluidleitende Verbindung bzw. Verbindungsleitung (Gaszuleitung) mit einer externen Gasquelle verbunden.

Vorzugsweise umfasst das zweite Kompartiment den Gasauslass. Vorzugsweise ist der Gasauslass im zweiten Kompartiment oder an der Decke des zweiten Kompartiments angeordnet. Dieser ist mit anderen Worten in diesem Fall an der Decke des zweiten Kompartiments angeordnet und über eine fluidleitende Verbindung bzw. Verbindungsleitung mit der Umgebung verbunden.

Vorzugsweise ist die Zugriffsöffnung in einer Wand des ersten oder des zweiten Kompartiments angeordnet, insbesondere in einer Wand, die den Reaktorinnenraum nach außen begrenzt. Durch diese Anordnung wird ein direkter Zugang zum jeweiligen Kompartiment ermöglicht, ohne dass benachbarte Kompartimente geöffnet oder demontiert werden müssen.

Das erste und das zweite Kompartiment sind vorzugsweise einseitig offen und weisen demnach eine offene Seite und eine der offenen Seite gegenüberliegende geschlossene Seite (mit anderen Worten an Ober- und Unterseite) sowie eine (laterale) geschlossene Seitenwand auf.

In einer bevorzugten Ausführungsform ist der Gasverteiler ein statischer Begaser, vorzugsweise ein Begaserring wie ein Ringsparger, eine Sinterplatte, ein spiralförmiges gelochtes Rohr oder eine Lochplatte. Bei statischen Begasern erfolgt die Gasdispergierung durch Öffnungen wie Poren oder Bohrungen im Gasverteiler. Zum Einsatz kommen Ringsparger, Sinterplatten, gelochte Rohre oder Lochplatten, die wirksame Gasverteiler zur Gleichverteilung des Gases über den Querschnitt darstellen, sodass ein hoher Gasgehalt im Reaktor erzielbar und die Stoffaustauschfläche vergrößert ist. Es ist zudem keine Zufuhr zusätzlicher äußerer Energie erforderlich. Durch die Verwendung von Sinterplatten lassen sich sehr feine Blasen erzeugen.

Gemäß einer weiteren bevorzugten Ausgestaltung der Erfindung ist das Trennelement horizontal angeordnet und ist eine perforierte Platte, ein Trenngitter, ein Siebeinbau wie ein Siebboden und/oder ein Metalldrahtgewebe. Der Siebboden enthält vorzugsweise eine Vielzahl gleich großer Öffnungen. Er besteht vorzugsweise entweder aus Metall (Lochblech, Drahtgewebe, Metallgitter oder Metallstäben), Kunststoff, Gummi verschiedener Härten oder Seidengaze. Die Größe der Öffnungen wird als Maschenweite w bezeichnet und definiert den Siebschnitt.

In einer ebenfalls bevorzugten Ausführungsform umfasst das Trennelement ein Material, das aus einer Gruppe von druckbaren Kunststoffen, vorzugsweise aus Polypropylen, PP, Polyethylenterephthalat, PET, glykolisiertes Polyethylenterephthalat, PETG, Polycarbonat, PC, Polyamid, PA, oder Polymilchsäure, PLA, ausgewählt ist. Hierdurch kann in einfacher Weise anwendungsspezifisch ein entsprechendes Trennelement bereitgestellt werden. Dabei ist PET recyclebar. Polycarbonat ist transparent, sodass eine verbesserte Ausnutzung des eingestrahlten Lichts ermöglicht wird. Polymilchsäure ist biologisch abbaubar, da es aus nachwachsenden Rohstoffen (Maisstärke) hergestellt wird.

In einer bevorzugten Ausgestaltung beträgt ein Durchmesser, ein Äquivalentdurchmesser oder ein hydraulischer Durchmesser der Öffnungen des Trennelements 1 mm bis 3 mm, vorzugsweise 1,5 mm bis 2,5 mm und besonders bevorzugt 1,9 mm bis 2,1 mm. Der Durchmesser und der Äquivalentdurchmesser sind vorzugsweise ein arithmetischer Mittelwert, sofern sich die Durchmesser und Äquivalentdurchmesser der Öffnungen unterscheiden. Im Falle von kreisrunden Querschnitten der Öffnungen wird der Durchmesser verwendet, der dem geometrischen Durchmesser entspricht. Der Äquivalentdurchmesser ist ein Maß für die Größe einer unregelmäßig geformten Öffnung. Er berechnet sich aus dem Vergleich einer Eigenschaft der unregelmäßigen Öffnung mit einer Eigenschaft einer regelmäßig geformten Öffnung. Vorzugsweise ist der Äquivalentdurchmesser der Durchmesser eines projektionsflächengleichen Kreises. Im Fall von einem Trennelement mit unregelmäßigen Öffnungen, die folglich einen veränderlichen Verlauf, eine veränderliche Länge (die nicht einer Dicke des Trennelements entspricht) und einen veränderlichen Querschnitt umfasst, eignet sich auch der hydraulische Durchmesser als entsprechende Dimension für die Öffnungen.

Im Fall eines Siebeinbaus oder eines Metalldrahtgewebes beträgt die Maschenweite w bevorzugt 1 mm bis 3 mm, vorzugsweise 1,5 mm bis 2,5 mm und besonders bevorzugt 1,9 mm bis 2,1 mm. Besonders geeignet ist eine Maschenweite w von 2 mm. Im Falle des Siebeinbaus oder des Metalldrahtgewebes bezieht sich der Durchmesser der Öffnungen auf eine Maschenweite w des Siebeinbaus oder des Metalldrahtgewebes, wobei die Maschenweite w der lichte Abstand zwischen zwei benachbarten Kett- oder Schussdrähten ist, in der Mitte der Maschenweite w gemessen. Die Längsrichtung einer Gewebebahn wird Kette genannt, die Querrichtung heißt Schuss. Mit der Angabe von Maschenweite w, Drahtdurchmesser und Bindung sind Quadrat- und Rechteckmaschengewebe eindeutig beschrieben. Der Drahtdurchmesser bezieht sich immer auf den Drahtdurchmesser vor dem Verweben. Die Maschenweite w wird am einfachsten über das Messreihenverfahren ermittelt. Dabei wird die Anzahl von Teilungen d für eine bestimmte Länge festgestellt. Diese Länge wird durch die Anzahl der Teilungen d dividiert, wodurch man den Mittelwert der Teilungen d erhält. Zieht man davon den Drahtdurchmesser ab, erhält man die Maschenweite w. Um einen arithmetischen Mittelwert der Maschenweite w zu erhalten, müssen so viele Teilungen d gemessen werden, wie notwendig sind, um einen zuverlässigen statistischen Wert zu erhalten. Bei Maschenweiten w zwischen 16 mm und 1 mm sollten 10 Teilungen d, bei kleineren Maschenweiten w bis 0,1 mm 20 Teilungen d abgezählt werden.

Gemäß einer weiteren bevorzugten Ausgestaltung der Erfindung beträgt ein Anteil einer offenen Fläche des Trennelements 40 % bis 60 %, vorzugsweise 45 % bis 55 % und besonders bevorzugt 49 % bis 53 % einer Gesamtfläche des Trennelements. Der Begriff "offene Fläche" des Trennelements bezeichnet vorzugsweise denjenigen Anteil der Gesamtfläche des Trennelements, der nicht von Material bedeckt ist und der somit für einen Durchtritt von Fluid, insbesondere Gas oder Flüssigkeit, durch das Trennelement zur Verfügung steht. Mit anderen Worten beträgt eine freie Fläche des Trennelements 40 % bis 60 %, vorzugsweise 45 % bis 55 % und besonders bevorzugt 49 % bis 53 %. In einer besonders bevorzugten Ausführungsform beträgt der Anteil der offenen Fläche 51 %. Die freie Fläche bzw. der freie Querschnitt oder die offene Fläche ist der prozentuale Durchlass der geöffneten Fläche. Mit anderen Worten wird hierdurch der Anteil der durchströmbaren Fläche des Trennelements an der gesamten Fläche des Trennelements (also der Summe aus Fläche der Öffnungen und der geschlossenen Fläche) beschrieben. Als offene oder freie Fläche A₀ wird im Falle des Siebeinbaus oder des Metalldrahtgewebes der prozentuale Anteil aller Öffnungen des Trennelements an der gesamten Fläche des Trennelements bezeichnet. A₀ beeinflusst den Durchflusswiderstand. Mit anderen Worten berechnet sich der Anteil der freien Fläche erhalten durch A₀ = (w/(w+d))² * 100 %.

In einer ebenfalls bevorzugten Ausführungsform umfasst der Photobioreaktor ferner zumindest ein drittes Kompartiment und eine Vielzahl von Trennelementen, wobei das dritte Kompartiment zwischen dem ersten und dem zweiten Kompartiment angeordnet ist und zwischen dem dritten Kompartiment und dem ersten Kompartiment sowie zwischen dem dritten Kompartiment und dem zweiten Kompartiment jeweils ein Trennelement der Vielzahl von Trennelementen angeordnet ist. Mit anderen Worten, ist das dritte Kompartiment dabei vorzugsweise zwischen dem ersten und dem zweiten Kompartiment angeordnet. Jeweils eines der Trennelemente ist zwischen dem dritten und dem ersten Kompartiment sowie zwischen dem dritten und dem zweiten Kompartiment positioniert.

In einer weiteren bevorzugten Ausführungsform umfasst der Photobioreaktor ferner eine Anzahl n dritter Kompartimente und eine Anzahl (n+1) von Trennelementen mit n ≥ 1, wobei das dritte Kompartiment zwischen dem ersten und dem zweiten Kompartiment angeordnet ist und wobei zwischen jedem dritten Kompartiment und direkt benachbart angeordneten anderen Kompartimenten jeweils ein Trennelement der Anzahl (n+1) von Trennelementen angeordnet ist. n ist dabei eine natürliche Zahl. Die ersten, zweiten und dritten Kompartimente bilden demzufolge vorzugsweise den zumindest einen Teil des Reaktorinnenraums, vorzugsweise den gesamten Reaktorinnenraum, aus. Die ersten, zweiten und dritten Kompartimente sind fluidleitend miteinander verbunden. Mit anderen Worten ist jedes der Anzahl n dritter Kompartimente zwischen dem ersten und dem zweiten Kompartiment angeordnet. Beispielsweise umfasst der Photobioreaktor zwei, drei oder mehr dritte Kompartimente, die allesamt zwischen erstem und zweitem Kompartiment angeordnet sind. Mit anderen Worten ist ferner jedes dritte Kompartiment direkt zwischen zwei anderen Kompartimenten, die sowohl das erste, zweite und/oder ein weiteres drittes Kompartiment sein können, angeordnet, wobei jedes dritte Kompartiment durch zwei Trennelemente begrenzt wird bzw. direkt zwischen zwei Trennelementen angeordnet ist. Im Fall nur eines (n=1) dritten Kompartiments liegen folglich zwei (1+1) Trennelemente vor, von denen eines der Trennelemente zwischen erstem und drittem Kompartiment und das andere der Trennelemente zwischen dem dritten Kompartiment und dem zweiten Kompartiment angeordnet ist. Bei Vorhandensein von zwei (n=2) dritten Kompartimenten liegen drei (2+1) Trennelemente vor, wobei das gegenüber dem vorherigen Beispiel zusätzliche dritte der drei Trennelemente nicht mehr direkt zwischen einem dritten Kompartiment und einem des ersten und zweiten Kompartiments angeordnet ist. Dieses zusätzlich dritte Trennelement ist vielmehr direkt zwischen den zwei dritten Kompartimenten und nur indirekt zwischen erstem und zweitem Kompartiment angeordnet. Die angrenzend bzw. direkt benachbart zu dem dritten Kompartiment angeordneten anderen Kompartimente können das erste oder zweite Kompartiment, aber auch ein weiteres drittes Kompartiment sein. Liegen beispielsweise drei (n=3) dritte Kompartimente vor, grenzen dabei zwei der dritten Kompartimente direkt an das erste bzw. zweite Kompartiment an, wobei das dritte Kompartiment nun zwischen den anderen beiden dritten Kompartimenten direkt angrenzend bzw. benachbart angeordnet ist. Durch die fluidleitende Verbindung durchströmt das zugeführte Gas sämtliche Kompartimente. Mit anderen Worten ist der Behälter des Photobioreaktors vorzugsweise ein modular aufgebauter Behälter, mit dem ersten Kompartiment und dem zweiten Kompartiment jeweils als Endstücke und vorzugsweise mindestens einem zwischen dem ersten Kompartiment und dem zweiten Kompartiment angeordneten weiteren dritten Reaktorkompartiment. Die Anordnung aller Kompartimente erfolgt dabei in vertikaler Richtung, sodass eine Reihe aus alternierend gestapelten Kompartimenten und Trennelementen vorliegt. Demnach umfasst der Photobioreaktor vorzugsweise mindestens drei, besonders bevorzugt mehr als drei, durch die Kompartimente in Verbindung mit den Trennelementen ausgebildete Kultivierungskammern, wobei die mindestens drei Kultivierungskammern durch vorzugsweise stoffschlüssiges, form- und/oder kraftschlüssiges Zusammenfügen des ersten Kompartiments mit zumindest einem dritten Kompartiment und vorzugsweise stoffschlüssiges, form- und/oder kraftschlüssiges Zusammenfügen des zweiten Kompartiments mit dem zumindest einen dritten Kompartiment ausgebildet sind. Bei Vorhandensein von mehr als einem dritten Kompartiment sind die dritten Kompartimente jeweils zumindest mit einem anderen dritten Kompartiment verbunden. Bei drei dritten Kompartimenten werden diese ebenfalls zwischen dem ersten und zweiten Kompartiment angeordnet, wobei nicht jedes dritte Kompartiment direkt an das erste und zweite Kompartiment angrenzt bzw. direkt benachbart zu diesen angeordnet ist. Bei zwei dritten Kompartimenten ist jedes der beiden dritten Kompartimente nur mit einem von dem ersten und zweiten Kompartiment verbunden sowie jeweils mit dem verbleibenden dritten Kompartiment. Bei drei dritten Kompartimenten ist das dritte der dritten Kompartimente nur mit den ersten beiden der dritten Kompartimente verbunden, also mit anderen Worten direkt zwischen den beiden anderen dritten Kompartimenten angeordnet, aber weiterhin indirekt zwischen dem ersten und dem zweiten Kompartiment angeordnet. Die einzelnen Kultivierungskammern des Photobioreaktors stehen miteinander in Fluidverbindung durch die Trennelemente. Hierdurch ist eine Skalierung durch Numbering-up möglich.

n ist vorzugsweise größer als 1 und kleiner als 16, vorzugsweise größer als 5 und kleiner als 13 und besonders bevorzugt größer als 8 und kleiner als 11. Besonders bevorzugt ist n gleich 9 oder gleich 10. Hierdurch wird einerseits der Photobioreaktor in geeigneter Weise hin zu größeren Maßstäben skaliert, wobei andererseits bei einer übermäßigen Anzahl an Kompartimenten der hydrostatische Druck zu hohe Werte annimmt und negativen Einfluss auf das Material des Photobioreaktors ausübt.

Vorzugsweise sind das erste und das zweite Kompartiment jeweils einseitig offen und das dritte Kompartiment ist vorzugsweise beidseitig offen. Das erste und das zweite Kompartiment weisen demnach jeweils eine offene Seite und eine der offenen Seite gegenüberliegende geschlossene Seite (mit anderen Worten an Ober- und Unterseite) sowie eine (laterale) geschlossene Seitenwand auf. Das dritte Kompartiment weist demnach zwei einander gegenüberliegende offene Seiten (mit anderen Worten an Ober- und Unterseite) sowie eine (laterale) geschlossene Seitenwand auf.

In einer weiteren bevorzugten Ausführungsform umfasst der Photobioreaktor einen Hohlstab zur Verteilung von Gas, der sich innerhalb des Reaktorinnenraums ausgehend von dem oberen Bereich zu dem Gasverteiler im unteren Bereich erstreckt und fluidleitend mit dem Gasverteiler verbunden ist. Der Vorteil der Luftleitung durch den oberen Bereich besteht in der Minimierung der Gefahr des Flüssigkeitsrückstaus oder des Auslaufens im Falle eines Druckluftausfalls der externen Gasquelle. Vorzugsweise erstreckt sich der Hohlstab durch einen Deckel des Behälters, der vorzugsweise das zweite Kompartiment ist, hin zu einem Boden des Behälters, der vorzugsweise das erste Kompartiment ist. Der Hohlstab ist vorzugsweise ebenfalls mit der externen Gasquelle verbunden.

In einer ebenfalls bevorzugten Ausführungsform ist das Trennelement mit einer Innenwand des Reaktorinnenraums verbunden.

Vorzugsweise ist das Trennelement mit dem Hohlstab verbunden. Mit anderen Worten ist das Trennelement an dem inneren vertikalen Hohlstab befestigt. Der Hohlstab dient gleichzeitig als Gaszuführungsleitung durch den oberen Bereich des Bioreaktors hin zum unteren Bereich, wo das Gas durch den Gasverteiler austritt und als Halteelement für das Trennelement, was die Montage vereinfacht.

Gemäß einer ebenfalls bevorzugten Ausführungsform sind die Kompartimente zylinderförmig und/oder sind die Kompartimente kugelähnlich. Mit anderen Worten weisen die Kompartimente im ersten Fall vorzugsweise einen kreisförmigen Querschnitt auf und bilden im Verbund eine zylindrische Säule aus. Im zweiten Fall bildet ein Verbund aus kugelähnlichen Kompartimenten vorzugsweise einen Kugelsegmentreaktor aus. Auch eine Kombination beider Kompartimentarten ist möglich. Kugelähnlich bedeutet vorliegend, dass ein dreidimensionaler Körper vorliegt, der gekrümmt ist. Dabei besitzt er vorzugsweise die erste Dimension in der horizontalen Richtung, die der Stelle mit dem höchsten Wert dieser Dimension entspricht, also mit anderen Worten dem breitesten Bereich entspricht. Dieser erste Wert entspricht in dem Verhältnis von Höhe zu Durchmesser dem Durchmesser. Ferner besitzt er vorzugsweise die zweite Dimension in der vertikalen Richtung, die der Stelle mit dem höchsten Wert dieser Dimension entspricht, also mit anderen Worten dem höchsten Bereich entspricht. Dieser zweite Werte entspricht in dem Verhältnis von Höhe zu Durchmesser der Höhe. Mit anderen Worten ist der Querschnitt des kugelähnlichen Kompartiments senkrecht zu einer horizontalen Richtung vorzugsweise ein Oval, also eine ebene rundliche konvexe Figur, vorzugsweise mit einer Symmetrieachse in vertikaler Richtung und/oder in horizontaler Richtung. Vorzugsweise umfasst die ovale Figur eine Ellipse. Der dreidimensionale Körper des kugelähnlichen Kompartiments ist also ein Ovoid. Für den Fall, dass das kugelähnliche Kompartiment Symmetrieachsen aufweist, werden die erste und/oder zweite Dimension vorzugsweise entlang dieser Symmetrieachsen bestimmt, begrenzt durch Außenkanten des Kompartiments. Ergänzend sei klargestellt, dass unter einem kugelähnlichen Kompartiment vorzugsweise ein ovoider, also eiförmiger, dreidimensionaler Körper verstanden wird, dessen größter horizontaler Abstand dem Durchmesser und dessen größter vertikaler Abstand der Höhe entspricht. Der Querschnitt senkrecht zur horizontalen Richtung ist dabei vorzugsweise oval oder elliptisch. Diese Definition dient der eindeutigen geometrischen Beschreibung und schließt vorzugsweise symmetrische wie auch asymmetrische Ausführungsformen ein.

Im Falle von kugelähnlichen Kompartimenten beträgt das Verhältnis von Höhe zu Durchmesser bevorzugt 1 bis 1,5, vorzugsweise 1 bis 1,2 und besonders bevorzugt 1,1.

Vorzugsweise beträgt der Durchmesser der zylinderförmigen Kompartimente mehr als 0,2 m und/oder der Durchmesser der kugelähnlichen Kompartimente mehr als 0,28. Die Gametophyten von Sphagnum weisen Durchmesser von etwa 0,6 cm bis 3 cm auf. Bei der entsprechend besagten Dimensionierung der Kompartimente kann eine vorteilhafte Umlaufströmung (Zirkulationsströmung) und demzufolge eine ständige Suspendierung der Gametophyten sichergestellt werden, sodass Licht- und Nährstoffverfügbarkeit für das Torfmoos gleichmäßig verteilt sind.

In einer besonders bevorzugten Ausgestaltung der Erfindung sind die Kompartimente integral einstückig ausgebildet. Alternativ weisen die Kompartimente vorzugsweise miteinander korrespondierende Flansche auf und sind miteinander durch Flanschverbindung verbunden. Mit anderen Worten umfasst der Photobioreaktor im ersten Fall vorzugsweise mehrere integrale Kompartimente, die also einstückig miteinander ausgebildet sind und die jeweils Kultivierräume bzw. -volumina ausbilden, zwischen denen sich das Trennelement zur Ausbildung der einzelnen Kultivierkammern befindet. Das Trennelement ist vorzugsweise innerhalb eines Innenraums der integralen Kompartimente angeordnet. Somit werden die einzelnen Kultivierkammern vorzugsweise durch die Anordnung des Trennelements bereitgestellt, wobei der Photobioreaktor bzw. der Behälter selbst dabei vorzugsweise integral einstückig ausgebildet ist. Mit anderen Worten liegt vorzugsweise ein integraler Behälter vor, der ursprünglich einen zusammenhängenden Innenraum umfasst, der dem Reaktorinnenraum entspricht, der durch das in ihm angeordnete Trennelement in mehrere Teil-Innenräume unterteilt ist, die jeweils ein Kompartiment darstellen und fluidleitend miteinander verbunden sind. Mit anderen Worten ist der Photobioreaktor bzw. der Behälter bzw. der zumindest eine Teil des Reaktorinnenraums im zweiten Fall vorzugsweise modular aufgebaut. Dabei werden mehrere separate (nicht integrale) Kompartimente durch Flanschverbindung miteinander verbunden. Jedes Kompartiment weist vorzugsweise zumindest einen Flansch auf, der mit dem Flansch eines anderen Kompartiments zur Verbindung dieser beiden Kompartimente korrespondiert. Eine Flanschverbindung besteht aus zwei gegenüberliegenden Bauteilen, die (aufgrund ihrer Form) Flansche genannt werden und durch mehrere Schrauben verbunden sind. Vorzugsweise befindet sich eine Dichtung zwischen den Flanschen.

Vorzugsweise ist das Trennelement innerhalb eines verbundenen Bereichs zweier benachbarter Kompartimente also mit anderen Worten innerhalb eines von beiden Kompartimenten ausgebildeten Hohlraums angeordnet. Alternativ ist das Trennelement vorzugsweise Teil der Flanschverbindung. Mit anderen Worten ist das Trennelement vorzugsweise zwischen den separaten Kompartimenten angeordnet, wobei vorzugsweise jeweils zwischen Trennelement und jedem der Kompartimente eine Dichtung vorgesehen ist. Durch die Verschraubung der Flansche von beiden Kompartimenten wird entsprechend Kraft auf das dazwischen angeordnete Trennelement ausgeübt und dieses fixiert und abgedichtet. Das Trennelement kann ebenfalls ein Art Flansch umfassen mit Bohrungen, die zu den Bohrungen der Flansche der beiden Kompartimente korrespondieren, sodass sich Schrauben auch durch die Bohrungen des Trennelements erstrecken.

Vorzugsweise ist das Trennelement in einem Flansch eines der beiden Kompartimente angeordnet bzw. montiert.

Besonders vorteilhaft ist eine Kombination aus integralen Kompartimenten und von oben nach unten geführtem Hohlstab, da das Trennelement aus Montagegründen ohnehin vorzugsweise von oben eingeführt wird.

Gemäß einer ebenfalls bevorzugten Ausführungsform umfassen die Kompartimente Acrylglas und/oder Polycarbonat, PC. Polymethylmethacrylat (Kurzzeichen PMMA, auch Acrylglas) ist ein transparenter thermoplastischer Kunststoff. Mit anderen Worten umfassen die Kompartimente Materialen der genannten Art. Vorzugsweise bestehen die Kompartimente aus Acrylglas, welches ein robustes, gut lichtdurchlässiges und recyclebares Material ist. Alternativ umfassen die Kompartimente Polycarbonat, einen gut lichtdurchlässigen und kostengünstigen Polymerwerkstoff. Besonders bevorzugt bestehen die Kompartimente aus zumindest einem der genannten Materialien.

Vorzugsweise sind die Innenwände der Kompartimente glatt. Dafür weisen die Innenwände eine Oberfläche mit einer absoluten Rauigkeit k von weniger als 0,03 mm auf. Die absolute Rauigkeit k ist vorzugsweise kleiner als 0,02 mm, bevorzugt kleiner als 0,01 mm und besonders bevorzugt kleiner als 0,007 mm. Rauigkeitsmessungen von Oberflächen sind in Form von Linienprofilen und als flächige, dreidimensionale Rauigkeitsmessungen möglich. Die Messung erfolgt vorzugsweise mittels Profilometer-Sonde nach DIN EN ISO 25178. Die Auswertung profilometrischer Analysen ist in den Normen DIN EN ISO 4287 für Linienprofile und DIN EN ISO 25178 für dreidimensionale Rauheitsmessungen von Oberflächen geregelt. In den Normen werden auch die Parameter der Rauigkeitsmessung festgelegt. Die ISO 25178 definiert den oberflächen bezogenen Rauigkeitswert Sₐ bzw. Rₐ über das arithmetische Mittel der Topographiehöhe z(x,y). Ein geeignetes optisches Messverfahren ist die konfokale Mikroskopie. Der Oberflächenrauhigkeitswert Sa kann kleiner als 0,04 mm, kleiner als 0,03 mm und kleiner als 0,01 mm sein. Hierdurch wird in Wandnähe die Wirbelbildung verringert sowie der Widerstandsbeiwert verringert.

Bevorzugt ist der Gasverteiler zu einer Zufuhr von Gas mit 0,1 vvm bis 1 vvm, vorzugsweise 0,15 vvm bis 0,7 vvm, besonders bevorzugt von 0,2 vvm bis 0,4 vvm ausgebildet. Besonders geeignet ist eine Begasungsrate von 0,3 vvm. 1 vvm steht für 1 Luftvolumen pro Volumen des Mediums pro Minute. Es ist eine Einheit, die zur Beschreibung der Belüftungsrate bzw. Begasungsrate in Bioreaktoren verwendet wird, insbesondere im Zusammenhang mit mikrobieller Fermentation autotropher Mikroorganismen und Zellkulturen. Durch die Wahl der Begasungsrate ist die Strömungsform beeinflussbar. Bei niedrigen Begasungsrate tritt eine homogene Strömung mit enger Größenverteilung der Gasblasen auf. Steigt die Begasungsrate, stellt sich eine heterogene Strömung ein, bei der sich größere Blasenaggregate ausbilden, die schneller aufsteigen. Vorliegend ist bei der Torfmoosproduktion nicht der Stofftransport von der gasförmigen Phase zur flüssigen Phase geschwindigkeitsbestimmend. Zudem erfolgt der Aufstieg bevorzugt in einem mittleren Bereich. Somit begünstig eine heterogene Strömung die für die Torfmoosproduktion gewünschten Vorgänge. Entsprechende Betrachtungen gelten in analoger Weise für eine Leerrohrgeschwindigkeit, sodass diese entsprechend auszuwählen ist. Die Gasleerrohrgeschwindigkeit beträgt vorzugsweise 0,1 m/min bis 1,43 m/min, vorzugsweise 0,14 m/min bis 1,003 m/min und besonders bevorzugt 0,156 bis 0,573 m/min. Besonders geeignet ist eine Leerrohrgeschwindigkeit von 0,3 m/min. Die Messung von Volumenstrom zur Bestimmung der Begasungsrate bzw. Gasleerrohrgeschwindigkeit erfolgt vorzugsweise mittels eines Rotameters (Schwebekörper-Durchflussmesser) oder vorzugsweise mittels eines Massendurchflussreglers mit kalorimetrischem Durchflussmesser oder Coriolis-Massendurchflussmesser (mass flow controller).

Vorzugsweise ist der Gasverteiler zu einer solchen Zufuhr von Gas ausgebildet, dass sich eine heterogene Strömung ausbildet. Bei höheren Gasbelastungen verschwindet die gleichförmige Verteilung der Gasblasen. Es entsteht eine stark turbulente Strömungsstruktur. In diesem heterogenen Strömungsbereich kommt es zur Bildung von Großblasen bzw. Blasenagglomeraten, die mit hoher Geschwindigkeit bevorzugt in der Säulenmitte aufsteigen. Dadurch kommt es teilweise zu so heftigen Zirkulationsströmungen, dass kleine Blasen in der Nähe der Behälterwand wieder nach unten transportiert werden.

In einer weiteren bevorzugten Ausgestaltung der Erfindung umfasst der Photobioreaktor eine Rückführung, die an ihrem einen Ende fluidleitend mit dem Gasauslass und an ihrem anderen Ende fluidleitend mit dem Gasverteiler verbunden ist. Mit anderen Worten ist die Rückführung vorzugsweise so ausgebildet, dass aus dem Gasauslass entnommenes Abgas zu dem Gasverteiler rückführbar ist. Die Rückführung kann direkt mit dem Gasverteiler verbunden sein oder mittels einer Gaszuleitung. Das durch den Gasverteiler im unteren Bereich zugeführte Gas steigt in der Flüssigkeit auf und verlässt diese je nach Intensität der Stoffübertragung und der chemischen Umsetzung mehr oder weniger verbraucht. Immer dann, wenn das Abgas noch wertvolle Einsatzstoffe in hoher Konzentration enthält, bietet sich eine teilweise Rückführung in den Reaktor an. Zudem werden hierdurch Emissionen von unverbrauchtem CO₂ reduziert.

Vorzugsweise umfasst der Photobioreaktor eine Vielzahl von Zugriffsöffnung und jedes Kompartiment umfasst jeweils eine der Vielzahl von Zugriffsöffnung.

Ein zweiter Aspekt der vorliegenden Offenbarung betrifft ein Verfahren zur submersen Produktion von Torfmoos, welches mittels eines erfindungsgemäßen Photobioreaktors durchgeführt wird. Das Verfahren umfasst ein Zugeben von dem mineralischen Nährmedium und den Torfmoos-Gametophyten in den zumindest einen Teil des Reaktorinnenraums mittels der Zugriffsöffnung sowie ein Bestrahlen des zumindest einen Teils des Reaktorinnenraums mit Licht. Ferner umfasst das Verfahren ein Zuleiten wenigstens eines Gases zum Gasverteiler und Zuführen des Gases in den zumindest einen Teil des Reaktorinnenraums mittels des Gasverteilers sowie ein Ableiten des Abgases aus dem Behälter mittels des Gasauslasses. Nach entsprechender Zeit erfolgt zudem ein Entnehmen von produziertem Torfmoos. Das Bestrahlen, das Begasen mit Luft und das Ableiten des Abgases können vorzugsweise zeitgleich erfolgen.

In bevorzugter Ausgestaltung der Erfindung erfolgt das Zuführen des Gases derart, dass sich eine radiale Zirkulationsströmung ausbildet, wobei das Gas vorzugsweise mit 0,1 vvm bis 1 vvm, vorzugsweise 0,15 vvm bis 0,7 vvm, besonders bevorzugt von 0,2 vvm bis 0,4 vvm zugeführt wird. Besonders geeignet ist eine Begasungsrate von 0,3 vvm. Mit anderen Worten wird Gas vorzugsweise mit einer solchen Begasungsrate oder Leerrohrgeschwindigkeit zugeführt, dass sich eine radiale Zirkulationsströmung ausbildet. Vorzugsweise wird das Gas mit einer Gasleerrohrgeschwindigkeit von 0,1 m/min bis 1,43 m/min, vorzugsweise 0,14 m/min bis 1,003 m/min und besonders bevorzugt 0,156 bis 0,573 m/min zugeführt. Besonders geeignet ist eine Leerrohrgeschwindigkeit von 0,3 m/min. Vorzugsweise erfolgt das Zuführen des Gases derart, dass sich eine heterogene Strömung ausbildet.

In ebenfalls bevorzugter Ausgestaltung ist das zugeführte Gas Luft oder beträgt ein CO₂-Gehalt des zugeführten Gases 0,04 Volumen-% bis 10 Volumen-%, vorzugsweise 0,4 Volumen-% bis 4 Volumen-% und besonders bevorzugt 1 Volumen-% bis 2 Volumen-%.

Vorzugsweise erfolgt das Bestrahlen des Reaktorinnenraums mit natürlichem Umgebungslicht und/oder künstlichem Licht unter Verwendung einer Lichtquelle.

Ein dritter Aspekt betrifft ein System zur submersen Produktion von Torfmoos umfassend den erfindungsgemäßen Photobioreaktor. Das System umfasst ferner einen mit dem Gasverteiler mittels einer Gaszuleitung verbundenen Kompressor als externe Gasquelle zur Bereitstellung von komprimierter Luft (Druckluft) für den Gasverteiler zur Durchmischung des Mediums, als Transportmedium für einen Kohlenstoffdioxidstrom und zum Abtransport von Sauerstoff aus dem Nährmedium. Die Gaszuleitung verfügt über ein Absperrventil zum Öffnen und Schließen der Gaszuleitung für eine Begasung des Photobioreaktors.

Die Gaszuleitung umfasst bevorzugt ein Druckminderer-Regelventil zur Einstellung des Betriebsdrucks und eine Druckminderer-Regelung zur Regelung des Betriebsdrucks. Die Gaszuleitung umfasst bevorzugt ein Rotameter-Nadelventil zur Einstellung des Luftvolumenstroms der Druckluft für den Photobioreaktor sowie eine Rotameter-Anzeigeskala mit Schwebekörper für eine Anzeige des Luftvolumenstroms.

Die Gaszuleitung umfasst bevorzugt einen Filter zum Schutz vor Kontaminationseintrag sowie eine Gaswaschflasche mit Wasser zur Luftbefeuchtung, um Wasseraustrag aus dem Photobioreaktor durch Begasung zu minimieren.

Das System umfasst ferner bevorzugt eine mit dem Gasauslass verbundene Gasableitung. Die Gasableitung umfasst vorzugsweise eine Wasserabscheidung für Abgas aufweisend einen Schlangenkühler zur Kondensation von Wasserdampf, um eine mit dem Abgas ausgetragene Wassermenge in den Photobioreaktor zurückzuführen sowie ein Thermostat zur Einstellung der Kühltemperatur.

Vorzugsweise umfasst die Gasableitung einen Feuchtigkeitsabscheider zum Schutz eines nachgeschalteten Filters vor Feuchtigkeit und Biomassepartikeln, wobei der nachgeschaltete Filter zum Schutz vor Kontaminationseintrag durch zurückgeführtes Wasser dient.

In bevorzugter Ausgestaltung der Erfindung umfasst das System eine externe CO₂-Quelle zur CO₂-Anreicherung von der durch den Gasverteiler zugeführten komprimierten Luft. Die CO₂-Quelle ist mittels einer Gasleitung mit der Gaszuleitung verbunden. Die Gasleitung umfasst bevorzugt ein Druckminderer-Regelventil zur Einstellung des Betriebsdrucks und eine Druckminderer-Regelung zur Regelung des Betriebsdrucks. Die Gasleitung umfasst bevorzugt ein Rotameter-Nadelventil zur Einstellung des CO₂-Volumenstroms für den Photobioreaktor sowie eine Rotameter-Anzeigeskala mit Schwebekörper für eine Anzeige des CO₂-Volumenstroms. Die Gasleitung umfasst optional ein Manometer zur prozessnahen Anzeige und Anzeige von Druckverlust über lange Leitungen.

Die Gasleitung umfasst bevorzugt einen Filter zum Schutz vor Kontaminationseintrag sowie eine Gaswaschflasche mit Wasser zur Gasbefeuchtung, um Wasseraustrag aus dem Photobioreaktor durch Begasung zu minimieren.

### Kurzbeschreibung der Figuren

Die Erfindung wird nachfolgend anhand eines Ausführungsbeispiels und dazugehöriger Zeichnungen näher erläutert. Die Figuren zeigen:
Fig. 1 Rohrleitungs- und Instrumentenfließschema eines Systems mit einem Photobioreaktor gemäß einer ersten Ausführung der Erfindung,
Fig. 2 Rohrleitungs- und Instrumentenfließschema eines Systems mit einem Photobioreaktor gemäß einer zweiten Ausführung der Erfindung,
Fig. 3 eine schematische Darstellung eines erfindungsgemäßen Verfahrens zur submersen Produktion von Torfmoos gemäß einer Durchführungsform.

### Detaillierte Beschreibung der Erfindung

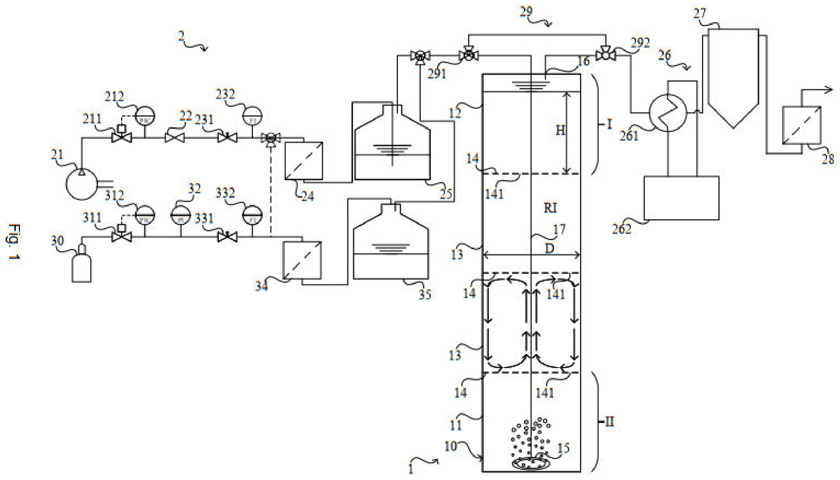

Fig. 1 zeigt ein Verfahrens- und Anlagenschema eines Photobioreaktors 1 gemäß einer ersten Ausführung der Erfindung sowie ein System 2 zur submersen Produktion von Torfmoos umfassend den Photobioreaktor 1. Der Photobioreaktor 1 umfasst einen Behälter 10 mit einem Reaktorinnenraum RI mit zumindest einem Teil des Reaktorinnenraums RI zur Aufnahme von einem mineralischen Nährmedium und Torfmoos-Gametophyten, wobei der zumindest eine Teil vorliegend den gesamten Reaktorinnenraum RI ausmacht. Der Behälter 10 weist ein erstes Kompartiment 11 und ein über dem ersten Kompartiment 11 angeordnetes zweites Kompartiment 12 auf sowie vorzugsweise zwei (Anzahl n=2) dritte Kompartimente 13 und vorzugsweise drei Trennelemente 14, wobei die dritten Kompartimente 13 zwischen dem ersten und dem zweiten Kompartiment 11, 12 angeordnet sind. Zwischen jedem dritten Kompartiment 13 und direkt benachbart angeordneten anderen Kompartimenten ist jeweils ein Trennelement 14 der drei Trennelemente 14 angeordnet.

Dabei bilden die Kompartimente 11, 12, 13 den Reaktorinnenraum RI aus, wobei die Kompartimente - zur Einkopplung von eingestrahltem Licht in den zumindest einen Teil des Reaktorinnenraums - vorzugsweise aus Acrylglas bestehen und im Wesentlichen transparent sind. Ein Verhältnis von Höhe zu Durchmesser jedes Kompartiments beträgt vorliegend exemplarisch 1,8. Die Trennelemente 14 verfügen über eine Vielzahl von Öffnungen 141 zur fluidleitenden Verbindung von sowohl jeweils benachbart angeordneten Kompartimenten 11, 12, 13 sowie den Kompartimenten 11, 12, 13 in ihrer Gesamtheit. Der Behälter 10 umfasst ferner eine nicht dargestellte Zugriffsöffnung zur Zugabe und Entnahme von Nährmedium und Gametophyten zum Reaktorinnenraum (und zur Reinigung). Zudem umfasst der Behälter 10 einen in einem unteren Bereich II des Reaktorinnenraums RI mittig angeordneten Gasverteiler 15, vorzugsweise in Form eines Ringspargers (Begaserring) mit einer Vielzahl von nicht dargestellten Gasöffnungen zur Zufuhr von Gas in den Reaktorinnenraum RI entlang eines begasten Teilbereichs einer Photobioreaktorquerschnittsfläche, und einen in einem oberen Bereich I des Reaktorinnenraums RI angeordneten Gasauslass 16 zur Abfuhr von Abgas aus dem Behälter 10. Der Gasauslass 16 ist im zweiten Kompartiment 12 angeordnet. Die Offenbarung ist nicht auf die vorstehend beschriebenen Ausführungsformen beschränkt und kann auch ohne beziehungsweise mit einer beliebigen Anzahl von dritten Kompartimenten 13 verwirklicht sein.

Mit anderen Worten liegen in vertikaler Richtung gestapelte Kompartimente 11, 12, 13 vor, wobei das erste Kompartiment 11 ein Endstück ist und einen Boden des Behälters 10 ausbildet und wobei das zweite Kompartiment 12 ein Endstück ist und einen Deckel des Behälters 10 ausbildet. Zwischen Boden und Deckel sind dann die dritten Kompartimente 13 angeordnet. Zwischen unmittelbar benachbarten Kompartimenten 11, 12, 13 ist ein Trennelement 14 angeordnet, sodass mehrere Kultivierungsräume bzw. -kammern des Reaktorinnenraums **RI** ausgebildet werden, die miteinander in fluidleitender Verbindung stehen, aber ein Hindernis für Partikel und jeweils in einem Kultivierungsraum ausgebildete Strömungsfelder darstellen.

Die Kompartimente 11, 12, 13 sind vorzugsweise zylinderförmig und bilden einen Säulenreaktor aus. Sie weisen einen Durchmesser D sowie eine Höhe H auf, die in der Fig. 1 lediglich die qualitativen Verhältnisse abbilden. Das Verhältnis von Höhe zu Durchmesser beträgt vorzugsweise 1,8. Der Durchmesser D der zylinderförmigen Kompartimente 11, 12, 13 beträgt vorzugsweise 0,2 m.

Rein schematisch ist dargestellt, wie das Zuführen von Gas mittels des Gasverteilers 15 erfolgt. Dabei wird ein Gasstrom in der flüssigen Phase in Form von Blasen dispergiert. Die Maße und Dimensionen sind rein schematisch dargestellt und deuten die teilflächige Begasung bzgl. der gesamten Photobioreaktorquerschnittsfläche an. Das Gas wird entlang des begasten Teilbereichs der Photobioreaktorquerschnittsfläche zugeführt und entlang eines verbleibenden unbegasten Teilbereichs der Photobioreaktorquerschnittsfläche erfolgt keine Zufuhr von Gas. Zudem ist erkennbar, dass die Blasen durch Koaleszenz und Agglomeration nach oben hin größer werden. Der Gasverteiler 15 ist vorzugsweise in einem zentralen Bereich der Photobioreaktorquerschnittsfläche des ersten Kompartiments 11 angeordnet, sodass die Zufuhr von Gas zentral-axial erfolgt. Die ausgebildete radiale Zirkulationsströmung ist schematisch durch Pfeile, die den Richtungsverlauf der Strömung andeuten, in einem dritten Kompartiment 13 dargestellt. Ein Verhältnis der Fläche des begasten Teilbereichs zu einer Fläche des unbegasten Teilbereichs beträgt vorzugsweise in etwa 25 %. Der Gasverteiler 15 ist zu einer Zufuhr von Gas mit 0,3 vvm ausgebildet.

Die Kompartimente 11, 12, 13 sind vorliegend integral einstückig ausgebildet. Die Trennelemente 14 sind innerhalb eines Innenraums der integralen Kompartimente 11, 12, 13 angeordnet. Somit werden die einzelnen Kultivierräume bzw. -kammern durch die Anordnung der Trennelemente 14 bereitgestellt, wobei der Photobioreaktor 1 bzw. der Behälter 10 selbst dabei integral einstückig ausgebildet ist. Mit anderen Worten liegt ein integraler Behälter 10 vor, der ursprünglich einen zusammenhängenden Innenraum umfasst, der dem Reaktorinnenraum RI entspricht, der durch die in ihm angeordneten Trennelemente 14 in mehrere Teil-Innenräume unterteilt ist, die jeweils ein Kompartiment 11, 12, 13 darstellen und fluidleitend miteinander verbunden sind. Die Trennelemente 14 können beispielsweise von oben in den Behälter 10 eingebracht werden. Die Trennelemente 14 sind vorliegend als Trenngitter ausgebildet und bestehen vorzugsweise aus Polypropylen. Die Öffnungen des Trenngitters sind quadratisch oder rund und weisen eine Maschenweite oder einen Durchmesser von 2 mm auf. Im Falle quadratischer Öffnungen entspricht die Maschenweite der Kantenlänge der Öffnungen. Im Falle runder Öffnungen entspricht der Durchmesser dem Kreisdurchmesser der Öffnung. Ein Anteil einer offenen bzw. freien Fläche des Trennelements 14 beträgt 51 % der Gesamtfläche des Trennelements 14.

Der Photobioreaktor 1 kann eine nicht dargestellte Lichtquelle umfassen oder auch auf einer Freifläche angeordnet werden, um Umgebungslicht bzw. Sonnenlicht ausgesetzt zu werden.

Der Photobioreaktor 1 umfasst ferner vorzugsweise einen Hohlstab 17 zur Verteilung von Gas, der sich innerhalb des Reaktorinnenraums **RI** ausgehend von dem oberen Bereich I zu dem Gasverteiler 15 erstreckt. Der Hohlstab erstreckt sich durch einen Deckel des Behälters 10, der vorliegend das zweite Kompartiment 12 ist, hin zu einem Boden des Behälters 10, der vorliegend das erste Kompartiment 11 ist. Die Trennelemente 14 sind zur Montage mit dem Hohlstab 17 verbunden. Die Offenbarung ist nicht auf die vorstehend beschriebenen Ausführungsformen beschränkt und kann auch ohne den Hohlstab 17 verwirklicht sein.

Der Photobioreaktor umfasst vorzugsweise eine Rückführung 29, die an ihrem einen Ende mittels eines 3-Wege-Ventils 292 fluidleitend mit dem Gasauslass 16 und an ihrem anderen Ende mittels eines Mischventils 291 fluidleitend mit dem Gasverteiler 15 durch den Hohlstab 17 verbunden ist. Mit anderen Worten ist die Rückführung 29 so ausgebildet, dass aus dem Gasauslass 16 entnommenes Abgas zu dem Gasverteiler 15 rückführbar ist. Die Offenbarung ist nicht auf die vorstehend beschriebenen Ausführungsformen beschränkt und kann auch ohne die Rückführung 29 verwirklicht sein.

Das System 2 umfasst ferner einen mit dem Gasverteiler 15 mittels einer Gaszuleitung verbundenen Kompressor 21 zur Bereitstellung von komprimierter Luft (Druckluft) für den Gasverteiler 15 zur Durchmischung des Mediums, als Transportmedium für einen Kohlenstoffdioxidstrom und zum Abtransport von Sauerstoff aus dem Nährmedium. Die Gaszuleitung verfügt über ein Absperrventil 22 zum Öffnen und Schließen der Gaszuleitung für eine Begasung des Photobioreaktors 1. Die Offenbarung ist nicht auf die vorstehend beschriebenen Ausführungsformen beschränkt und kann auch ohne den Kompressor 21 verwirklicht sein.

Die Gaszuleitung umfasst ferner ein Druckminderer-Regelventil 211 zur Einstellung des Betriebsdrucks und eine Druckminderer-Regelung 212 zur Regelung des Betriebsdrucks. Die Gaszuleitung umfasst ein Rotameter-Nadelventil 231 zur Einstellung des Luftvolumenstroms der Druckluft für den Photobioreaktor 1 sowie eine Rotameter-Anzeigeskala 232 mit Schwebekörper für eine Anzeige des Luftvolumenstroms. Die Offenbarung ist nicht auf die vorstehend beschriebenen Ausführungsformen beschränkt und kann auch ohne das Druckminderer-Regelventil 211 verwirklicht sein.

Die Gaszuleitung umfasst einen Filter 24 zum Schutz vor Kontaminationseintrag sowie eine Gaswaschflasche 25 mit Wasser zur Luftbefeuchtung, um Wasseraustrag aus dem Photobioreaktor 1 durch Begasung zu minimieren. Die Offenbarung ist nicht auf die vorstehend beschriebenen Ausführungsformen beschränkt und kann auch ohne Filter 24 verwirklicht sein.

Das System 2 umfasst ferner eine mit dem Gasauslass 16 verbundene Gasableitung. Die Gasableitung umfasst eine Wasserabscheidung 26 für Abgas aufweisend einen Schlangenkühler 261 zur Kondensation von Wasserdampf, um eine mit dem Abgas ausgetragene Wassermenge in den Photobioreaktor 1 zurückzuführen sowie ein Thermostat 262 zur Einstellung der Kühltemperatur. Die Offenbarung ist nicht auf die vorstehend beschriebenen Ausführungsformen beschränkt und kann auch ohne Gasableitung verwirklicht sein.

Die Gasableitung umfasst zudem einen Feuchtigkeitsabscheider 27 zum Schutz eines nachgeschalteten Filters 28 vor Feuchtigkeit und Biomassepartikeln, wobei der nachgeschaltete Filter 28 zum Schutz vor Kontaminationseintrag durch zurückgeführtes Wasser dient.

Das System 1 weist eine CO₂-Quelle in Form einer CO₂-Gasflasche 30 zur CO₂-Anreicherung der von dem Gasverteiler 15 zugeführten komprimierten Luft auf. Die CO₂-Quelle ist mittels einer Gasleitung mit der Gaszuleitung verbunden. Die Gasleitung umfasst ein Druckminderer-Regelventil 311 zur Einstellung des Betriebsdrucks und eine Druckminderer-Regelung 312 zur Regelung des Betriebsdrucks. Die Gasleitung umfasst ein Rotameter-Nadelventil 331 zur Einstellung des CO₂-Volumenstroms für den Photobioreaktor 1 sowie eine Rotameter-Anzeigeskala 332 mit Schwebekörper für eine Anzeige des CO₂-Volumenstroms. Die Gasleitung umfasst ein Manometer 32 zur prozessnahen Anzeige und Anzeige von Druckverlust über lange Leitungen. Die Offenbarung ist nicht auf die vorstehend beschriebenen Ausführungsformen beschränkt und kann auch ohne CO₂-Gasflasche 30 verwirklicht sein.

Die Gasleitung umfasst ferner einen Filter 34 zum Schutz vor Kontaminationseintrag sowie eine Gaswaschflasche 35 mit Wasser zur CO₂-Befeuchtung, um Wasseraustrag aus dem Photobioreaktor 1 durch Begasung zu minimieren. Der Filter 34 und die Gaswaschflasche 35 können in einer Variante des Systems 1 auch entfallen, indem die Zuführung des CO₂-Volumenstroms aus der CO₂-Quelle zum Druckluftstrom schon vor dem Filter 24 erfolgt. Dies ist anhand der gestrichelt dargestellten Leitung mit dem gestrichelt dargestellten Mischventil angedeutet. Die Offenbarung ist nicht auf die vorstehend beschriebenen Ausführungsformen beschränkt und kann auch ohne Filter 34 verwirklicht sein.

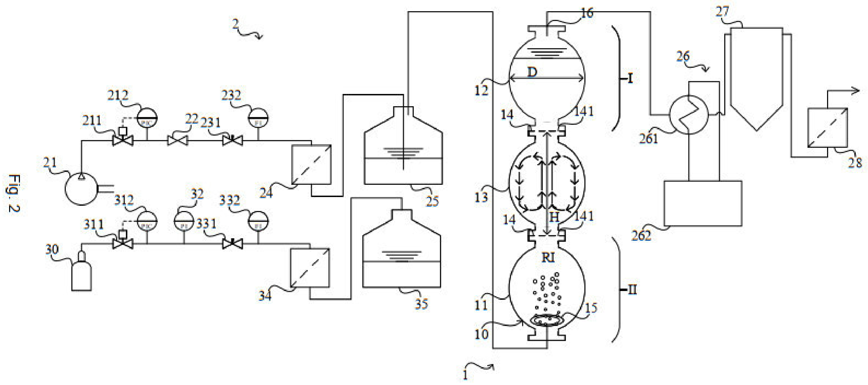

Fig. 2 zeigt ein Verfahrens- und Anlagenschema eines Photobioreaktors 1 gemäß einer zweiten Ausführung der Erfindung sowie ein System 2 zur submersen Produktion von Torfmoos umfassend den Photobioreaktor 1. Elemente und Komponenten, die denjenigen der ersten Ausführung aus Fig. 1 entsprechen, werden nicht erneut beschrieben, insofern ist die entsprechende Beschreibung aus Fig. 1 auf diese Komponenten anzuwenden.

Der Behälter 10 weist ein erstes Kompartiment 11 und ein über dem ersten Kompartiment 11 angeordnetes zweites Kompartiment 12 auf sowie ein (Anzahl n=1) drittes Kompartiment 13 und zwei Trennelemente 14, wobei das dritte Kompartimente 13 zwischen dem ersten und dem zweiten Kompartiment 11, 12 angeordnet ist. Zwischen dem dritten Kompartiment 13 und dem benachbart angeordneten ersten bzw. zweiten Kompartiment 11, 12 ist jeweils ein Trennelement 14 angeordnet. Dabei bilden die Kompartimente 11, 12, 13 den Reaktorinnenraum RI aus, wobei die Kompartimente 11, 12, 13 - zur Einkopplung von eingestrahltem Licht in den zumindest einen Teil des Reaktorinnenraums - aus Acrylglas bestehen und im Wesentlichen transparent sind.

Mit anderen Worten liegen in vertikaler Richtung gestapelte Kompartimente 11, 12, 13 vor, wobei das erste Kompartiment 11 ein Endstück ist und einen Boden des Behälters 10 ausbildet und wobei das zweite Kompartiment 12 ein Endstück ist und einen Deckel des Behälters 10 ausbildet. Zwischen Boden und Deckel ist dann das dritte Kompartiment 13 angeordnet. Zwischen unmittelbar benachbarten Kompartimenten 11, 12, 13 ist ein Trennelement 14 angeordnet, sodass mehrere Kultivierungsräume bzw. -kammern des Reaktorinnenraums **RI** ausgebildet werden, die miteinander in fluidleitender Verbindung stehen, aber ein Hindernis für Partikel und jeweils in einem Kultivierungsraum ausgebildete Strömungsfelder darstellen. Die Offenbarung ist nicht auf die vorstehend beschriebenen Ausführungsformen beschränkt und kann auch ohne beziehungsweise mit einer beliebigen Anzahl von dritten Kompartimenten 13 verwirklicht sein.

Die Kompartimente 11, 12, 13 sind vorliegend vorzugsweise kugelähnlich. Kugelähnlich bedeutet vorliegend, dass ein dreidimensionaler Körper vorliegt, der gekrümmt ist. Die Kompartimente 11, 12, 13 besitzen eine erste Dimension in einer horizontalen Richtung, die einer Stelle mit dem höchsten Wert dieser Dimension entspricht, also mit anderen Worten einem breitesten Bereich entspricht. Dieser erste Werte entspricht in dem Verhältnis von Höhe zu Durchmesser dem Durchmesser D. Ferner besitzen die Kompartimente 11, 12, 13 eine zweite Dimension in einer vertikalen Richtung, die einer Stelle mit dem höchsten Wert dieser Dimension entspricht, also mit anderen Worten einem höchsten Bereich entspricht. Dieser zweite Werte entspricht in dem Verhältnis von Höhe zu Durchmesser der Höhe H. Im vorliegenden Fall beträgt das Verhältnis von Höhe zu Durchmesser vorzugsweise 1,1. Mit anderen Worten ist der Querschnitt des kugelähnlichen Kompartiments senkrecht zu einer horizontalen Richtung ein Oval, also eine ebene rundliche konvexe Figur, vorliegend mit einer Symmetrieachse in vertikaler Richtung und in horizontaler Richtung. Der dreidimensionale Körper der kugelähnlichen Kompartimente 11, 12, 13 ist also ein Ovoid. Der Durchmesser D der kugelähnlichen Kompartimente 11, 12, 13 beträgt 0,28 m.

Die Kompartimente 11, 12, 13 weisen vorzugsweise miteinander korrespondierende Flansche auf und sind miteinander durch Flanschverbindung verbunden. Mit anderen Worten ist der Photobioreaktor 1 bzw. der Behälter 10 modular aufgebaut. Dabei werden mehrere separate (nicht integrale) Kompartimente 11, 12, 13 durch Flanschverbindung miteinander verbunden. Die Trennelement 14 sind jeweils innerhalb eines verbundenen Bereichs zweier benachbarter Kompartimente 11, 12, 13 angeordnet.

Die beschriebenen Systeme 2 aus Fig. 1 und Fig. 2 umfassen folglich zwei erfindungsgemäße Ausführungsformen von Photobioreaktoren 1, wobei die Erfindung nicht auf diese Ausführungsformen beschränkt ist, die beispielhaft mit etwa 35 bzw. 40 Litern dem technischen Maßstab angehören und damit eine Möglichkeit zur Massenvermehrung von Torfmoos bieten. Sie bestehen aus Acrylglas, welches ein robustes, gut lichtdurchlässiges und recyclebares Material ist. Die Photobioreaktoren 1 gemäß der ersten und zweiten Ausführungsform bestehen aus einem segmentierten Behälter 10 bzw. aus mit Flanschen verbundenen Segmenten (Kompartimenten), was die Behältervergrößerung auf einfache Weise ermöglicht. Eine Produktionsvergrößerung ist somit nicht nur durch Numbering-Up, sondern auch durch Behältervergrößerung möglich. Hierdurch können einfach größere Volumina erreicht werden. Die Segmentierung erlaubt eine schrittweise Maßstabsvergrößerung. Unter Beibehaltung des Verhältnisses von Höhe zu Durchmesser wird ein weiteres Scale-Up durch größere Durchmesser und Behälterhöhen ermöglicht.

Die Reaktorinnenwände sind vorzugsweise glatt und die Reaktoren besitzen große Öffnungen, was die Reinigung und dadurch lange Wiederverwendbarkeit ermöglichen. Zudem betragen die Reaktordurchmesser 0,20 m (erste Ausführung) bzw. 0,28 m (zweite Ausführung), wodurch ein Verhaken und Anstauen der Gametophyten verhindert wird. Unterstützt wird die ständige Durchmischung der Biomasse in den Behältern 10 durch den Gaseintrag von unten, welcher im Bereich von 0 vvm bis 1 vvm regelbar ist. Dies ermöglicht einen regelbaren spezifischen Leistungseintrag, der zum Beispiel bei steigender Biomassekonzentrationen zur Durchmischung benötigt wird.

Die vollständige Flotation oder Sedimentation der Biomasse wird durch die horizontalen Trennelemente 14 verhindert, welche in regelmäßigen Abständen die Photobioreaktoren 1 segmentieren. So kann der Photobioreaktor 1 gemäß der zweiten Ausführung z. B. in drei Segmente und der Photobioreaktor 1 gemäß der ersten Ausführung in vier Segmente gegliedert werden. Die Trennelemente 14 bestehen bevorzugt aus Polypropylen mit Öffnungen mit einem Durchmesser von 2 mm und einer freien Fläche von 51%. Dies führt zur Rückhaltung von ausschließlich der Biomasse, nicht aber von Flüssig- und Gasphase. So kann die Vermischung der Feststoffphase teilweise von der Bewegung von Flüssig- und Gasphase entkoppelt werden. Die Trennelemente 14 sind im Fall des Photobioreaktors 1 gemäß der zweiten Ausführung an der Behälterinnenwand und im Fall des Photobioreaktors 1 gemäß der ersten Ausführung an dem inneren vertikalen Hohlstab 17 befestigt. Der Hohlstab 17 dient gleichzeitig als Luftleitung durch den Deckel des Photobioreaktors 1 hin zum Boden, wo die Luft durch den Ringsparger 15 austritt. Der Vorteil der Luftleitung durch den Deckel besteht in der Minimierung der Gefahr des Flüssigkeitsrückstaus oder Auslaufen im Falle eines Druckluftausfalls.

Für eine gleichmäßige Durchmischung sorgt zusätzlich die bevorzugte innovative Form des Photobioreaktors 1. Durch die kugelähnliche Geometrie kann die Biomasse während der Begasung von unten in Halbkreisen und ganzen Kreisen entlang der Photobioreaktorwand strömen, was wenig Scherkräfte und dennoch vollständige Suspendierung und Durchmischung erzeugt. Ein ähnliches Strömungsbild wird in den einzelnen Segmenten des zylindrischen Photobioreaktors durch die Segmente mit einem Verhältnis von Höhe zu Durchmesser von 1,8 erzeugt.

Dieser Reaktoraufbau ermöglicht mit 0,1 g Trockensubstanz pro Liter Reaktorvolumen und Kultivierungstag bis 0,2 g Trockensubstanz pro Liter Reaktorvolumen und Kultivierungstag hohe Biomasseproduktivitäten, die mit denen im Labormaßstab (abhängig vom Kultivierungsmedium) vergleichbar sind.

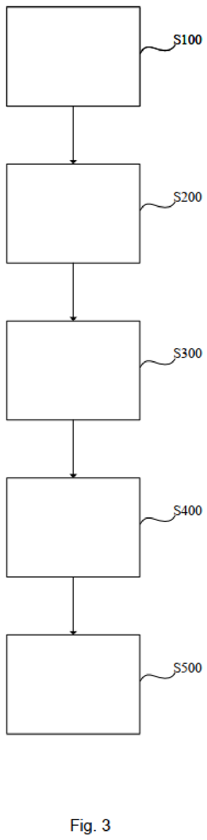

Fig. 3 ist eine schematische Darstellung eines erfindungsgemäßen Verfahrens zur submersen Produktion von Torfmoos gemäß einer Durchführungsform welches mittels eines erfindungsgemäßen Photobioreaktors 1 durchgeführt wird.

Das Verfahren umfasst einen Schritt S100, bei dem ein Zugeben von dem mineralischen Nährmedium und den Torfmoos-Gametophyten in den zumindest einen Teil des Reaktorinnenraums RI mittels der Zugriffsöffnung erfolgt.

Ferner umfasst das Verfahren einen Schritt S200, bei dem ein Bestrahlen des Reaktorinnenraums RI mit Licht erfolgt.

Zudem wird in Schritt S300 von dem Kompressor 21 bereitgestellte Druckluft, die optional nach Bedarf durch die CO₂-Quelle 30 mit CO₂ angereichert sein kann, zum Gasverteiler 15 zugeleitet und das Gas in den zumindest einen Teil des Reaktorinnenraums **RI** mittels des Gasverteilers 15 zugeführt.

Ferner umfasst das Verfahren einen Schritt S400, bei dem ein Ableiten des Abgases aus dem Behälter 10 mittels des Gasauslasses 16 erfolgt.

Nach entsprechender Zeit erfolgt zudem in Schritt S500 ein Entnehmen von produziertem Torfmoos.

Das Bestrahlen, das Begasen mit Luft und das Ableiten des Abgases können zeitgleich erfolgen.

Das Zuführen des Gases erfolgt vorliegend derart, dass sich eine radiale Zirkulationsströmung ausbildet, wobei das Gas vorzugsweise mit 0,3 vvm zugeführt wird. Mit anderen Worten wird Gas mit einer solchen Begasungsrate oder Leerrohrgeschwindigkeit zugeführt, dass sich eine radiale Zirkulationsströmung ausbildet. Wie beschrieben ist das zugeführte Gas Luft, die angereichert wurde, sodass ein CO₂-Gehalt des zugeführten Gases 15 bis 20 % beträgt. Das Bestrahlen des Reaktorinnenraums erfolgt durch Kombination aus natürlichem Umgebungslicht und künstlichem Licht unter Verwendung einer Lichtquelle.

### Bezugszeichenliste

- 1: Photobioreaktor
- 10: Behälter
- 11: erstes Kompartiment
- 12: zweites Kompartiment
- 13: drittes Kompartiment
- 14: Trennelement/Trenngitter
- 141: Öffnung
- 15: Gasverteiler/Ringsparger
- 16: Gasauslass
- 17: Hohlstab
- 2: System
- 21: Kompressor
- 211: Druckminderer-Regelventil
- 212: Druckminderer-Regelung
- 22: Absperrventil
- 231: Rotameter-Nadelventil
- 232: Rotameter-Anzeigeskala
- 24: Filter
- 25: Gaswaschflasche
- 26: Wasserabscheidung
- 261: Schlangenkühler
- 262: Thermostat
- 27: Feuchtigkeitsabscheider
- 28: Filter
- 29: Rückführung
- 291: Mischventil
- 292: 3-Wege-Ventil
- 30: CO₂-Gasflasche
- 311: Druckminderer-Regelventil
- 312: Druckminderer-Regelung
- 32: Manometer
- 331: Rotameter-Nadelventil
- 332: Rotameter-Anzeigeskala
- 34: Filter
- 35: Gaswaschflasche
- D: Durchmesser
- H: Höhe
- RI: Reaktorinnenraum
- I: oberer Bereich
- II: unterer Bereich
- S100: Zugeben von mineralischen Nährmedium und Torfmoos-Gametophyten
- S200: Bestrahlen des zumindest einen Teils des Reaktorinnenraums
- S300: Zuleiten und Zuführen wenigstens eines Gases
- S400: Ableiten des Abgases
- S500: Entnehmen von produziertem Torfmoos

## Patentansprüche

1. Photobioreaktor (1) zur submersen Produktion von Torfmoos umfassend einen Behälter (10) mit einem Reaktorinnenraum (RI) mit zumindest einem Teil des Reaktorinnenraums (RI) zur Aufnahme von einem mineralischen Nährmedium und Torfmoos-Gametophyten, der Behälter (10) aufweisend
ein erstes Kompartiment (11) und ein über dem ersten Kompartiment (11) angeordnetes zweites Kompartiment (12), wobei die Kompartimente (11, 12, 13) den zumindest einen Teil des Reaktorinnenraums (RI) ausbilden, im Wesentlichen transparent sind und ein Verhältnis von Höhe (H) zu Durchmesser (D) jedes Kompartiments 1 bis 2,6 beträgt,
ein zwischen dem ersten und dem zweiten Kompartiment (11, 12) angeordnetes Trennelement (14) aufweisend eine Vielzahl von Öffnungen (141) zur fluidleitenden Verbindung von den Kompartimenten (11, 12, 13),
eine verschließbare Zugriffsöffnung zur Zugabe und Entnahme von mineralischem Nährmedium und Torfmoos-Gametophyten in den bzw. aus dem zumindest einen Teil des Reaktorinnenraums (RI),
einen in einem unteren Bereich (II) des Reaktorinnenraums (RI) mittig angeordneten Gasverteiler (15) mit einer Vielzahl von Gasöffnungen zur Zufuhr von Gas in den zumindest einen Teil des Reaktorinnenraums (RI) entlang eines begasten Teilbereichs einer Photobioreaktorquerschnittsfläche, und
einen in einem oberen Bereich (I) des Reaktorinnenraums (RI) angeordneten Gasauslass (16) zur Abfuhr von Abgas aus dem Behälter (10).

2. Photobioreaktor (1) nach Anspruch 1, wobei ein Verhältnis von einer Fläche des begasten Teilbereichs zu einer Fläche des unbegasten Teilbereichs 0,1 bis 1, vorzugsweise 0,2 bis 0,7 und besonders bevorzugt 0,3 bis 0,5 beträgt.

3. Photobioreaktor (1) nach Anspruch 1 oder 2, wobei das erste Kompartiment (11) den unteren Bereich (II) des Reaktorinnenraums (RI) ausbildet und wobei das zweite Kompartiment (12) den oberen Bereich (I) des Reaktorinnenraums (RI) ausbildet.

4. Photobioreaktor (1) nach einem der vorhergehenden Ansprüche, wobei der Gasverteiler (15) ein statischer Begaser, vorzugsweise ein Begaserring, eine Sinterplatte, ein spiralförmiges gelochtes Rohr oder eine Lochplatte, ist.

5. Photobioreaktor (1) nach einem der vorhergehenden Ansprüche, wobei das Trennelement (14) horizontal angeordnet ist und eine perforierte Platte, ein Trenngitter, ein Siebeinbau und/oder ein Metalldrahtgewebe ist.

6. Photobioreaktor (1) nach einem der vorhergehenden Ansprüche, wobei das Trennelement (14) ein Material umfasst, das aus einer Gruppe von druckbaren Kunststoffen, vorzugsweise aus Polypropylen, PP, Polyethylenterephthalat, PET, glykolisiertes Polyethylenterephthalat, PETG, Polycarbonat, PC, Polyamid, PA oder Polymilchsäure, PLA, ausgewählt ist; und/oder wobei die Kompartimente (11, 12, 13) Acrylglas und/oder Polycarbonat umfassen.

7. Photobioreaktor (1) nach einem der vorhergehenden Ansprüche, wobei ein Durchmesser, ein Äquivalentdurchmesser oder ein hydraulischer Durchmesser der Öffnungen (141) des Trennelements (14) 1 mm bis 3 mm, vorzugsweise 1,5 mm bis 2,5 mm und besonders bevorzugt 1,9 mm bis 2,1 mm beträgt.

8. Photobioreaktor (1) nach einem der vorhergehenden Ansprüche, wobei ein Anteil einer offenen Fläche des Trennelements (14) 40 % bis 60 %, vorzugsweise 45 % bis 55 % und besonders bevorzugt 49 % bis 53 % einer Gesamtfläche des Trennelements (14) beträgt.

9. Photobioreaktor (1) nach einem der vorhergehenden Ansprüche, der Photobioreaktor (1) ferner umfassend eine Anzahl n dritter Kompartimente (13) und eine Anzahl (n+1) von Trennelementen (14) mit n ≥ 1, wobei das dritte Kompartiment (13) zwischen dem ersten und dem zweiten Kompartiment (11, 12) angeordnet ist und wobei zwischen jedem dritten Kompartiment (13) und direkt benachbart angeordneten anderen Kompartimenten (11, 12, 13) jeweils ein Trennelement (14) der Anzahl (n+1) von Trennelementen (14) angeordnet ist.

10. Photobioreaktor (1) nach einem der vorhergehenden Ansprüche, wobei der Photobioreaktor (1) einen Hohlstab (17) zur Verteilung von Gas umfasst, der sich innerhalb des Reaktorinnenraums (RI) ausgehend von dem oberen Bereich (I) zu dem Gasverteiler (15) im unteren Bereich (II) erstreckt und fluidleitend mit dem Gasverteiler (15) verbunden ist.

11. Photobioreaktor (1) nach einem der vorhergehenden Ansprüche, wobei die Kompartimente (11, 12, 13) zylinderförmig und/oder kugelähnlich sind.

12. Photobioreaktor (1) nach einem der vorhergehenden Ansprüche, wobei die Kompartimente (11, 12, 13) integral einstückig ausgebildet sind oder wobei die Kompartimente (11, 12, 13) miteinander korrespondierende Flansche aufweisen und miteinander durch Flanschverbindung verbunden sind.

13. Photobioreaktor (1) nach einem der vorhergehenden Ansprüche, wobei der Photobioreaktor (1) eine Rückführung (29) umfasst, die an ihrem einen Ende fluidleitend mit dem Gasauslass (16) und an ihrem anderen Ende fluidleitend mit dem Gasverteiler (15) verbunden ist.

14. Verfahren zur submersen Produktion von Torfmoos, welches mittels eines Photobioreaktors (1) nach einem der vorhergehenden Ansprüche durchgeführt wird, wobei das Verfahren umfasst:
Zugeben (S100) von dem mineralischen Nährmedium und den Torfmoos-Gametophyten in den zumindest einen Teil des Reaktorinnenraums (RI) mittels der Zugriffsöffnung;
Bestrahlen (S200) des zumindest einen Teils des Reaktorinnenraums (RI) mit Licht;
Zuleiten (S300) wenigstens eines Gases zum Gasverteiler (15) und Zuführen (S300) des Gases in den zumindest einen Teil des Reaktorinnenraums (RI) mittels des Gasverteilers (15);
Ableiten (S400) des Abgases aus dem Behälter (10) mittels des Gasauslasses (16); und
Entnehmen (S500) von produziertem Torfmoos.

15. Verfahren nach Anspruch 14, wobei das Zuführen (S300) des Gases derart erfolgt, dass sich eine radiale Zirkulationsströmung ausbildet, wobei das Gas vorzugsweise mit 0,1 vvm bis 1 vvm, vorzugsweise 0,15 vvm bis 0,7 vvm, besonders bevorzugt von 0,2 vvm bis 0,4 vvm zugeführt wird, und/oder wobei das Gas Luft ist oder wobei ein CO₂-Gehalt des zugeführten Gases 0,04 Volumen-% bis 10 Volumen-%, vorzugsweise 0,4 Volumen-% bis 4 Volumen-% und besonders bevorzugt 1 Volumen-% bis 2 Volumen-% beträgt.
